# EUROPÄISCHE PATENTANMELDUNG

(11) **EP 0 606 824 A1**
(43) Veröffentlichungstag der Anmeldung: **20.07.1994**
(21) Anmeldenummer: 93810906.3
(22) Anmeldetag: 24.12.1993
(51) Int. Cl.: C07D 295/18, A61K 31/495

(54) **Substituierte Dialkylthioether**

(30) Priorität: 15.01.1993 CH 115/93
(71) Anmelder: CIBA-GEIGY AG, CH-4002 Basel (CH)
(72) Erfinder: Ferrini, Pier Giorgio, Dr., CH-4102 Binningen (CH)

(57) **Zusammenfassung**

Substituierte Dialkylthioether der Formel I, 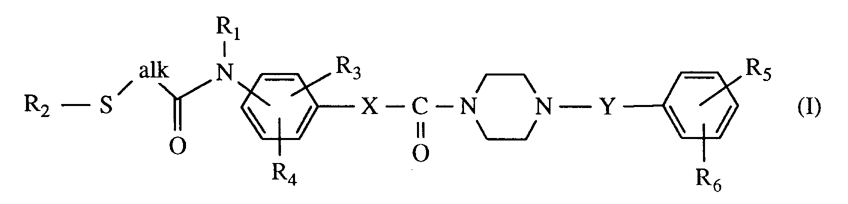
worin R₁ - R₆, alk, X und Y wie in der Beschreibung definiert sind, sowie Salze davon, weisen die Biosynthese von Interleukin-1 (IL-1) hemmende sowie analgetische Eigenschaften auf und können daher als Arzneimittelwirkstoffe verwendet werden. Sie werden in an sich bekannter Weise hergestellt.

## Beschreibung

Die Erfindung betrifft neue substituierte Dialkylthioether der Formel I, 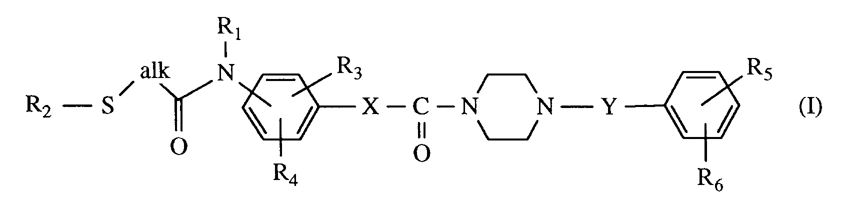
worin R₁ für Wasserstoff, Niederalkyl, Hydroxy-niederalkyl, Niederalkoxy-niederalkyl, Niederalkoxy-niederalkoxy-niederalkyl, Aryloxy-niederalkyl, Arylniederalkoxy-niederalkyl, N-Niederalkylamino-niederalkyl oder N,N-Diniederalkylamino-niederalkyl steht; R₂ Niederalkyl bedeutet, das durch einen Substituenten ausgewählt aus der Gruppe Amino, Acylamino, Carboxy und funktionell modifiziertes Carboxy substituiert ist und das gegebenenfalls weitere Substituenten ausgewählt aus der Gruppe Amino, Niederalkylamino, Diniederalkylamino, Acylamino, Oxo, Carboxy, funktionell modifiziertes Carboxy, Hydroxy, Niederalkoxy, Acyloxy und Halogen trägt; R₃ und R₄ unabhängig voneinander Wasserstoff, Niederalkyl, Halogen, Niederalkoxy oder Niederalkylthio bedeuten, R₅ und R₆ unabhängig voneinander für Wasserstoff, Niederalkyl, Halogen-niederalkyl, Niederalkoxy, Niederalkylthio, Halogen, Amino, Niederalkylamino, Diniederalkylamino oder Niederalkanoylamino stehen, alk Niederalkylen bedeutet, und X und Y unabhängig voneinander eine direkte Bindung, Niederalkylen oder Niederalkenylen bedeuten, und Salze davon, Verfahren zur Herstellung dieser Verbindungen, pharmazeutische Präparate, die diese Verbindungen enthalten, die Verwendung dieser Verbindungen zur therapeutischen Behandlung des menschlichen oder tierischen Körpers oder zur Herstellung pharmazeutischer Präparate.

Die Verbindungen der Formel I, welche ein asymmetrisches Kohlenstoffatom enthalten, können jeweils als Racemat oder als R- oder S-Enantiomer vorliegen. Die Erfindung bezieht sich auf alle diese Formen sowie z.B. auch auf Diastereomere und Gemische davon, die auftreten können, wenn zwei oder mehr asymmetrische Zentren im Molekül vorhanden sind.

Vor- und nachstehend sind unter niederen Resten und Verbindungen beispielsweise solche zu verstehen, die bis und mit 7, vorzugsweise bis und mit 4, Kohlenstoffatome (C-Atome) aufweisen.

Niederalkyl ist z.B. C₁-C₇-Alkyl, vorzugsweise C₁-C₄-Alkyl, wie insbesondere Methyl oder in zweiter Linie Ethyl, n-Propyl, Isopropyl oder n-Butyl, kann aber z.B. auch Isobutyl, sek.-Butyl, tert.-Butyl oder eine Pentyl-, Hexyl- oder Heptylgruppe sein.

Halogen-niederalkyl ist z.B. Trifluormethyl.

Halogen ist z.B. Chlor, Fluor oder Brom, kann aber auch für Iod stehen.

Hydroxy-niederalkyl und Niederalkoxy-niederalkyl tragen die Hydroxy- bzw. Niederalkoxygruppe vorzugsweise in höherer als der α-Stellung und bedeuten z.B. entsprechendes Hydroxy-C₂-C₄-alkyl oder C₁-C₄-Alkoxy-C₂-C₄-alkyl, wie z.B. 2-Hydroxyethyl, 2-Methoxyethyl, 2-Ethoxyethyl, 2-Propyloxyethyl, 2-Isopropyloxyethyl, 3-Methoxypropyl, 3-Ethoxypropyl, 3-Isopropyloxypropyl oder 4-Methoxybutyl.

Oxo steht für den zweiwertigen Substituenten =O.

Carbamoyl steht für die Gruppe -C(=O)-NH₂.

Niederalkylen ist z.B. Methylen, Ethylen, Propylen, Butylen oder Pentylen, aber z.B. auch Hexylen oder Heptylen; es umfasst ferner auch verzweigte Reste wie z.B. 1-Methyl-methylen, 1,1-Dimethyl-methylen oder 2-Methyl-1,3-propylen.

Niederalkylen als Bedeutung von alk in Formel I (bzw. von alk₁ in Formel Ia) ist bevorzugt C₁-C₄-Alkylen, insbesondere Methylen oder 1,2-Ethylen, und vor allen Dingen Methylen.

Niederalkylen als Bedeutung von alk₂ in Formel Ia, welches gegebenenfalls substituiert ist, steht bevorzugt für gegebenenfalls substituiertes C₁-C₄-Alkylen, und insbesondere für Methylen, 1-Methyl-methylen, 1,1-Dimethyl-methylen, 1-(Carboxy oder funktionell modifiziertes Carboxy)-methylen oder 1,2-Ethylen.

Niederalkenylen ist vorzugsweise C₂-C₇-Alkenylen, z.B. 1,3-Propenylen, 1,4-Butenylen, und insbesondere 1,2-Ethenylen.

Aryl ist z.B. Phenyl, das unsubstituiert oder durch einen oder zwei Substituenten aus der Gruppe bestehend aus Niederalkyl, Niederalkoxy, Hydroxy, Halogen und Trifluormethyl substituiert ist, und vor allen Dingen Phenyl.

Acylamino ist z.B. Niederalkanoylamino oder ein Rest -NH-W₁, worin W₁ für das über eine beliebige Carboxygruppe gebundene Radikal einer Aminosäure, oder ein Derivat davon, steht. Bevorzugt ist Acylamino Niederalkanoylamino.

Acyloxy ist z.B. Niederalkanoyloxy.

Funktionell modifiziertes Carboxy ist vorzugsweise Niederalkoxycarbonyl, Carbamoyl, N-Niederalkylcarbamoyl, N,N-Diniederalkylcarbamoyl oder Cyano, kann aber ferner z.B. auch für einen Rest -C(=O)-W₂, worin W₂ für das über eine Aminogruppe gebundene Radikal einer Aminosäure, oder eines Derivats davon, stehen.

Unter einer Aminosäure ist z.B. zu verstehen eine α- oder β-Aminosäure; insbesondere eine natürliche **α**-Aminosäure mit der L-Konfiguration, wie sie normalerweise in Proteinen vorkommt; oder ein Epimeres einer solchen Aminosäure, d.h. mit der unnatürlichen D-Konfiguration, oder das entsprechende D,L-Isomerengemisch, oder ein Homologes einer solchen Aminosäure, z.B. worin die Aminosäurenseitenkette um eine oder zwei Methylengruppen verlängert oder verkürzt ist, worin die Aminogruppe in β-Stellung vorliegt und/oder worin eine Methylgruppe durch Wasserstoff ersetzt ist, oder eine substituierte aromatische Aminosäure, worin der aromatische Rest 6 - 14 Kohlenstoffatome hat, z. B. ein substituiertes Phenylalanin oder Phenylglycin, worin der Phenylring etwa durch Niederalkyl, Niederalkoxy, Niederalkanoyloxy, Amino, Niederalkylamino, Diniederalkylamino, Niederalkanoylamino, Niederalkoxycarbonylamino; Arylmethoxycarbonylamino, worin Aryl vorzugsweise 6 - 14 Kohlenstoffatome hat, z. B. Benzyloxycarbonylamino oder 9-Fluorenylmethoxycarbonylamino; Halogen, Carboxy und/oder Nitro ein- oder mehrfach substituiert ist.

Von Derivaten dieser Aminosäuren wird im folgenden z.B. gesprochen, wenn freie Amino- oder Hydroxyfunktionen, vorzugsweise eine freie Aminofunktion, durch Acylreste wie oben definiert substituiert sind. Des weiteren, wenn in diesen Aminosäuren eine oder mehrere Carboxygruppen als funktionell modifiziertes Carboxy wie oben definiert vorliegen.

Salze von Verbindungen der Formel I sind insbesondere pharmazeutisch verwendbare Salze, z.B. (a) Säureadditionssalze mit geeigneten Mineralsäuren, wie Halogenwasserstoffsäuren, Schwefelsäure oder Phosphorsäure, z.B. Hydrochloride, Hydrobromide, Sulfate, Hydrogensulfate oder Phosphate, Salze mit geeigneten aliphatischen oder aromatischen Sulfonsäuren oder N-substituierten Sulfaminsäuren, z.B. Methansulfonate, Benzolsulfonate, p-Toluolsulfonate oder N-Cyclohexylsulfaminate (Cyclamate), oder Salze mit starken organischen Carbonsäuren, wie Niederalkancarbonsäuren oder gegebenenfalls ungesättigten oder hydroxylierten aliphatischen Dicarbonsäuren, z.B. Acetate, Oxalate, Malonate, Maleinate, Fumarate, Maleate, Tartrate oder Citronate.

Weiterhin sind auch z.B. (b) entsprechende Salze mit Basen umfasst, wie entsprechende Alkalimetall- oder Erdalkalimetallsalze, z. B. Natrium-, Kalium- oder Magnesiumsalze, pharmazeutisch verwendbare Uebergangsmetallsalze, wie Zink- oder Kupfersalze, oder Salze mit Ammoniak oder organischen Aminen, wie cyclischen Aminen, wie Mono-, Di- oder Triniederalkylaminen, wie Hydroxyniederalkylaminen, z. B. Mono-, Di- oder Trihydroxyniederalkylaminen, Hydroxyniederalkyl-niederalkyl-aminen oder Polyhydroxyniederalkylaminen. Cyclische Amine sind z. B. Morpholin, Thiomorpholin, Piperidin oder Pyrrolidin. Als Mononiederalkylamine kommen beispielsweise Ethyl- und t-Butylamin, als Diniederalkylamine beispielsweise Diethyl- und Diisopropylamin und als Triniederalkylamine beispielsweise Trimethyl- und Triethylamin in Betracht. Entsprechende Hydroxyniederalkylamine sind z. B. Mono-, Di- und Triethanolamin; Hydroxyniederalkvl-niederalkyl-amine sind z. B. N,N-Dimethylamino- und N,N-Diethylamino-ethanol.

Zur Isolierung oder Reinigung können auch pharmazeutisch ungeeignete Salze Verwendung finden. Zur therapeutischen Anwendung gelangen nur die pharmazeutisch verwendbaren, nicht-toxischen Salze, die deshalb bevorzugt sind.

Die Verbindungen der Formel I und ihre pharmazeutisch verwendbaren Salze weisen wertvolle pharmakologische Eigenschaften auf. Insbesondere zeigen sie ausgeprägte Hemmwirkung auf die Biosynthese von Interleukin-1 (IL-1). IL-1 gehört zur Klasse der proinflammatorischen Proteine und spielt beispielsweise bei der Prostaglandinsynthese, der Synthese neutraler Proteasen durch Fibroplasten, Synovialzellen und Chondrozyten, bei der Aktivierung von Endothelialzellen und bei der Induktion weiterer proinflammatorischer Zytokine, wie des **α**-Tumornekrosefaktors (TNF) und von Interleukin-6 (IL-6) eine wesentliche Rolle. Ferner regt es die Knochenresorption an, reguliert die Körpertemperatur von Warmblütern und reguliert u.a. die Entwicklung, Aktivierung, Differenzierung und Proliferation von Lymphozyten. Therapeutisch besonders wichtig ist die Hemmwirkung der Verbindungen der Formel I und ihrer pharmazeutisch verwendbaren Salze auf die Biosynthese von IL-1, TNF und IL-6. Diese kann in vitro beispielsweise an durch Lipopolysaccharide stimulierten (LPS-stimulierten) menschlichen Monozyten nach C. Rordorf-Adam et al., Drugs Exptl. Clin. Res. XV, 355-62(1989) im, Konzentrationsbereich ab etwa 0,1 µM und in vivo an der Maus anhand der Hemmung der LPS-induzierten Bildung von Serumamyloid P (SAP) bei einer ED₅₀ von etwa 1 bis 15 mg/kg p.o. und an der Ratte anhand der Senkung des durch LPS erzeugten künstlichen Fiebers bei einer ED₅₀ von etwa 0,05 bis 3,5 mg/kg p.o. gezeigt werden.

Aufgrund dieser Eigenschaften sind die Verbindungen der Formel I und ihre pharmazeutisch verwendbaren Salze vorzüglich geeignet zur therapeutischen Behandlung von Erkrankungen, bei denen eine übermässige Produktion von IL-1 eine ursächliche oder verschlimmernde Rolle spielt, wie entzündliche und degenerative Gelenkerkrankungen, beispielsweise rheumatoide Arthritis, Osteoarthrosis, psoriatische oder infektiöse Arthritis, das Reitersyndrom, Gicht und traumatische Arthritis, und andere akute oder chronische Entzündungen, beispielsweise entzündliche Darmerkrankungen, Meningitis, Hauterkrankungen, z.B. Psoriasis oder Pemphigus vulgaris, allergische Hautreaktionen, Atherosklerose und Autoimmunerkrankungen, wie Diabetes (Typ 1) und Thyroiditis.

Als weitere Erkrankungen, bei denen eine übermässige Produktion von IL-1 eine ursächliche oder verschlimmernde Rolle spielt, sind z.B. zu nennen: Knochenmetabolismus-Regulationsstörungen, z.B. Paget-Krankheit, Osteoporose, Periodontitis oder Malignitäten; oder endotoxischer Schock, z.B. verbunden mit Fieber, Hypotension und fulminantem Leberversagen.

Zusätzlich weisen die Verbindungen der Formel I und ihre pharmazeutisch verwendbaren Salze eine ausgeprägte analgetische Wirkung auf, die sich beispielsweise anhand der Hemmung des durch Phenyl-p-benzochinon ausgelösten Writhingsyndroms der Maus beispielsweise in einer an Hendershot und Forsaith, J. Pharmacol. Exp. Therap. 125, 237 (1959) angelehnten Versuchanordnung mit einer ED₅₀ von etwa 1 bis 30 mg/kg p.o. nachweisen lässt.

Die Verbindungen der Formel I und ihre pharniazeutisch verwendbaren Salze können dementsprechend auch als analgetische Arzneimittelwirkstoffe zur Behandlung von Schmerzzuständen unterschiedlicher Genese, insbesondere als periphere Analgetika, verwendet werden.

Bevorzugt betrifft die Erfindung die Verbindungen der Formel I, worin R₁ für Wasserstoff, Niederalkyl, Hydroxy-niederalkyl, Niederalkoxy-niederalkyl, Niederalkoxy-niederalkoxy-niederalkyl; Phenyloxy-niederalkyl oder Phenylniederalkoxy-niederalkyl, wobei in den beiden letztgenannten Resten die Phenylgruppe jeweils unsubstituiert oder durch Niederalkyl, Trifluormethyl, Niederalkoxy, Halogen und/oder Hydroxy substituiert ist; N-Niederalkylamino-niederalkyl oder N,N-Diniederalkylamino-niederalkyl steht; R₂ Niederalkyl bedeutet, das durch einen Substituenten ausgewählt aus der Gruppe gebildet aus Amino, Niederalkanoylamino, Carboxy, Niederalkoxycarbonyl, Carbamoyl, N-Niederalkylcarbamoyl, N,N-Diniederalkylcarbamoyl, Cyano und einem Rest -C(=O)-W₂, worin W₂ für das über eine Aminogruppe gebundene Radikal einer Aminosäure, oder ein Derivat davon, steht; substituiert ist und das gegebenenfalls weitere Substituenten ausgewählt aus der Gruppe gebildet aus Amino, Niederalkylamino, Diniederalkylamino, Niederalkanoylamino; einem Rest -NH-W₁, worin W₁ für das über eine beliebige Carboxygruppe gebundene Radikal einer Aminosäure, oder ein Derivat davon, steht; Oxo, Carboxy, Niederalkoxycarbonyl, Carbamoyl, N-Niederalkylcarbamoyl, N,N-Diniederalkylcarbamoyl, Cyano, Hydroxy, Niederalkoxy, Niederalkanoyloxy und Halogen trägt; R₃ und R₄ unabhängig voneinander Wasserstoff, Niederalkyl, Halogen, Niederalkoxy oder Niederalkylthio bedeuten, R₅ und R₆ unabhängig voneinander für Wasserstoff, Niederalkyl, Halogen-niederalkyl, Niederalkoxy, Niederalkylthio, Halogen, Amino, Niederalkylamino, Diniederalkylamino oder Niederalkanoylamino stehen, alk Niederalkylen bedeutet, und X und Y unabhängig voneinander eine direkte Bindung, Niederalkylen oder Niederalkenylen bedeuten, und Salze davon.

Besonders bevorzugt betrifft die Erfindung die Verbindungen der Formel I, worin R₁ für Wasserstoff, Niederalkyl, Hydroxy-niederalkyl, Niederalkoxy-niederalkyl, Niederalkoxy-niederalkoxy-niederalkyl, Phenyloxy-niederalkyl, N-Niederalkylamino-niederalkyl oder N,N-Diniederalkylamino-niederalkyl steht; R₂ Niederalkyl bedeutet, das durch einen Substituenten ausgewählt aus der Gruppe gebildet aus Amino, Carboxy, Niederalkoxycarbonyl, Carbamoyl, N-Niederalkylcarbamoyl, N,N-Diniederalkylcarbamoyl und einem Rest -C(=O)-W₂, worin W₂ für das über eine Aminogruppe gebundene Radikal einer natürlichen Aminosäure ausgewählt aus Glycin, Alanin, Valin, Leucin und Serin, oder einen Niederalkylester davon, steht; substituiert ist und das gegebenenfalls einen weiteren Substituenten ausgewählt aus der Gruppe gebildet aus Amino, Niederalkanoylamino; einem Rest -NH-W₁, worin W₁ für das über eine beliebige Carboxygruppe gebundene Radikal einer natürlichen Aminosäure ausgewählt aus Glutaminsäure, Asparaginsäure, Glycin, Alanin, Valin, Leucin und Serin, oder einen Niederalkylester davon, steht; Oxo, Carboxy, Niederalkoxycarbonyl, Carbamoyl, N,N-Diniederalkylcarbamoyl und Hydroxy trägt; R₃ und R₄ unabhängig voneinander Wasserstoff, Niederalkyl, Halogen, Niederalkoxy oder Niederalkylthio bedeuten, R₅ und R₆ unabhängig voneinander für Wasserstoff, Niederalkyl, Trifluormethyl, Niederalkoxy, Niederalkylthio, Halogen oder Diniederalkylamino stehen, alk Niederalkylen bedeutet, und X und Y unabhängig voneinander eine direkte Bindung, Niederaikylen oder Niederalkenylen bedeuten, und Salze davon.

Ganz besonders bevorzugt betrifft die Erfindung die Verbindungen der Formel I, worin R₁ für Wasserstoff, Niederalkyl, Hydroxy-niederalkyl oder Niederalkoxy-niederalkyl steht, R₂ Niederalkyl bedeutet, das durch einen Substituenten ausgewählt aus der Gruppe gebildet aus Amino, Carboxy, Niederalkoxycarbonyl und einem Rest -C(=O)-W₂, worin W₂ für das über die Aminogruppe gebundene Radikal der Aminosäure Glycin, oder einen Niederalkylester davon, steht, substituiert ist und das gegebenenfalls einen weiteren Substituenten ausgewählt aus der Gruppe gebildet aus Amino, Niederalkanoylamino; einem Rest -NH-W₁, worin W₁ für das über die γ-Carboxygruppe gebundene Radikal der Aminosäure L-Glutaminsäure, oder einen Niederalkylester davon, steht; Oxo, Carboxy und Niederalkoxycarbonyl trägt; R₃ und R₄ unabhängig voneinander Wasserstoff, Niederalkyl, Halogen, Niederalkoxy oder Niederalkylthio bedeuten, R₅ für Niederalkylthio, Chlor, Fluor oder Brom steht, R₆ Wasserstoff bedeutet, alk Methylen bedeutet, X für eine direkte Bindung oder 1,2-Ethenylen steht, und Y 1,2-Ethylen bedeutet, und pharmazeutisch verwendbare Salze davon.

Hervorzuheben unter den Verbindungen der Formel I sind die Verbindungen der Formel Ia, 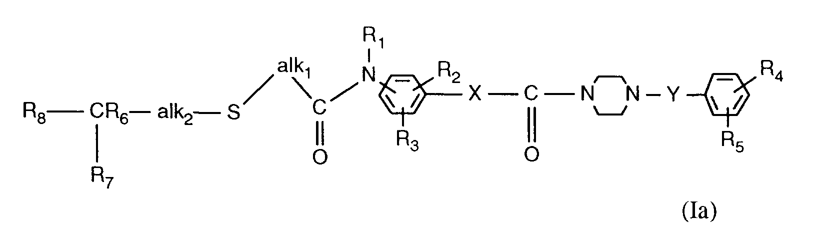
worin R₁ für Wasserstoff, Niederalkyl, Hydroxy-niederalkyl oder Niederalkoxyniederalkyl steht, R₂ und R₃ unabhängig voneinander Wasserstoff, Niederalkyl, Chlor, Fluor, Brom, Niederalkoxy oder Niederalkylthio bedeuten, R₄ und R₅ unabhängig voneinander Chlor, Fluor, Brom, Wasserstoff, Niederalkyl, Trifluormethyl, Niederalkoxy oder Niederalkylthio darstellen, R₆ für Wasserstoff steht, R₇ Wasserstoff, Amino, Niederalkanoylamino oder einen Rest -NH-W₁ bedeutet, worin W₁ für das über eine beliebige Carboxygruppe gebundene Radikal einer Aminosäure, oder ein Derivat davon, steht; oder R₆ und R₇ zusammen für Oxo stehen; R₈ Amino, Niederalkanoylamino, Carboxy, Niederalkoxycarbonyl, Carbamoyl, N-Niederalkylcarbamoyl, N,N-Diniederalkylcarbamoyl, Cyano oder einen Rest -C(=O)-W₂ bedeutet, worin W₂ für das über eine Aminogruppe gebundene Radikal einer Aminosäure, oder eines Derivats davon, steht; alk₁ für Niederalkylen steht, alk₂ eine direkte Bindung oder Niederalkylen, das unsubstituiert oder durch Carboxy, Niederalkoxycarbonyl, Carbamoyl, N-Niederalkylcarbamoyl, N,N-Diniederalkylcarbamoyl oder Cyano substituiert ist, bedeutet, X für eine direkte Bindung oder 1,2-Ethenylen steht, und Y eine direkte Bindung, Niederalkylen oder 1,2-Ethenylen bedeutet, und Salze davon.

Die Erfindung betrifft in erster Linie Verbindungen der Formel Ia, worin R₁ für Wasserstoff, Niederalkyl, Hydroxy-niederalkyl oder Niederalkoxyniederalkyl steht, R₂ und R₃ unabhängig voneinander Wasserstoff, Niederalkyl, Chlor, Fluor oder Brom bedeuten, R₄ Chlor, Fluor, Brom oder Niederalkylthio darstellt, R₅ Wasserstoff bedeutet, R₆ für Wasserstoff steht, R₇ Wasserstoff, Amino, Niederalkanoylamino oder einen Rest -NH-W₁ bedeutet, worin W₁ für das über eine beliebige Carboxygruppe gebundene Radikal einer natürlichen Aminosäure, oder eines Niederalkylesters davon, steht; oder R₆ und R₇ zusammen für Oxo stehen; R₈ Amino, Carboxy, Niederalkoxycarbonyl, Carbamoyl, N-Niederalkylcarbamoyl, N,N-Diniederalkylcarbamoyl oder einen Rest -C(=O)-W₂ bedeutet, worin W₂ für das über eine Aminogruppe gebundene Radikal einer natürlichen Aminosäure, oder eines Niederalkylesters davon, steht; alk₁ für Methylen steht, alk₂ eine direkte Bindung, Methylen, 1-Methyl-methylen, 1,1-Dimethyl-methylen, 1-(Carboxy oder Niederalkoxycarbonyl)-methylen oder 1,2-Ethylen bedeutet, X für eine direkte Bindung oder 1,2-Ethenylen steht, und Y 1,2-Ethylen bedeutet, und pharmazeutisch verwendbare Salze davon.

Die Erfindung betrifft vor allem Verbindungen der Formel Ia, worin R₁ Wasserstoff, C₁-C₄-Alkyl, Hydroxy-C₂-C₄-alkyl oder C₁-C₄-Alkoxy-C₂-C₄-alkyl bedeutet, R₂ und R₃ unabhängig voneinander Wasserstoff, Niederalkyl, Chlor, Fluor oder Brom bedeuten, R₄ in para-Stellung angeknüpft ist und Chlor, Fluor, Brom oder Niederalkylthio darstellt, R₅ Wasserstoff bedeutet, R₆ für Wasserstoff steht, R₇ Wasserstoff, Amino, Niederalkanoylamino oder einen Rest -NH-W₁ bedeutet, worin W₁ für das über eine beliebige Carboxygruppe gebundene Radikal einer natürlichen Aminosäure ausgewählt aus Glutaminsäure, Asparaginsäure, Glycin, Alanin, Valin, Leucin und Serin, oder eines Niederalkylesters davon, steht; oder R₆ und R₇ zusammen für Oxo stehen; R₈ Amino, Carboxy, Niederalkoxycarbonyl, Carbamoyl, N-Niederalkylcarbamoyl, N,N-Diniederalkylcarbamoyl oder einen Rest -C(=O)-W₂ bedeutet, worin W₂ für das über eine Aminogruppe gebundene Radikal einer natürlichen Aminosäure ausgewählt aus Glycin, Alanin, Valin, Leucin und Serin, oder eines Niederalkylesters davon, steht; alk₁ für Methylen steht, alk₂ eine direkte Bindung, Methylen, 1,1-Dimethyl-methylen, 1-(Carboxy oder Niederalkoxycarbonyl)-methylen oder 1,2-Ethylen bedeutet, X für eine direkte Bindung oder 1,2-Ethenylen steht, und Y 1,2-Ethylen bedeutet, und pharmazeutisch verwendbare Salze davon.

Die Erfindung betrifft insbesondere Verbindungen der Formel Ia, worin R₁ Wasserstoff, Methyl, Ethyl, 2-Hydroxyethyl oder 2-Isopropyloxyethyl bedeutet, R₂ und R₃ unabhängig voneinander Wasserstoff, Methyl, Chlor oder Fluor bedeuten, R₄ in para-Stellung angeknüpft ist und Chlor oder Brom darstellt, R₅ Wasserstoff bedeutet, R₆ für Wasserstoff steht, R₇ Wasserstoff, Amino, Niederalkanoylamino oder einen Rest -NH-W₁ bedeutet, worin W₁ für das über die γ-Carboxygruppe gebundene Radikal der Aminosäure L-Glutaminsäure, oder eines Niederalkylesters davon, steht; oder R₆ und R₇ zusammen für Oxo stehen; R₈ Amino, Carboxy, Niederalkoxycarbonyl oder einen Rest -C(=O)-W₂ bedeutet, worin W₂ für das über die Aminogruppe gebundene Radikal der Aminosäure Glycin, oder eines Niederalkylesters davon, steht; alk₁ für Methylen steht, alk₂ eine direkte Bindung, Methylen, 1,1-Dimethyl-methylen, 1-(Carboxy oder Niederalkoxycarbonyl)-methylen oder 1,2-Ethylen bedeutet, X für eine direkte Bindung oder 1,2-Ethenylen steht, und Y 1,2-Ethylen bedeutet, und pharmazeutisch verwendbare Salze davon.

Steht in einer Verbindung der Formel Ia R₈ für Amino oder Niederalkanoylamino, so bedeutet der Rest R₇ vorzugsweise Wasserstoff. Steht in einer Verbindung der Formel Ia alk₂ für eine direkte Bindung, so bedeutet der Rest R₇ vorzugsweise Wasserstoff.

In all den verschiedenen Gruppen von Verbindungen der Formel Ia, die oben erwähnt sind, sind jeweils diejenigen Verbindungen der Formel Ia weiter hervorzuheben, in denen R₇ für Niederalkanoylamino steht.

Die Erfindung betrifft namentlich die in den Beispielen genannten Verbindungen der Formel I und ihre Salze, insbesondere ihre pharmazeutisch verwendbaren Salze.

Das Verfahren zur Herstellung der Verbindungen der Formel I, welche die Verbindungen der Formel Ia voll mit einschliessen, beruht auf an sich bekannten Methoden und ist z.B. dadurch gekennzeichnet, dass man
a) eine Verbindung der Formel II, 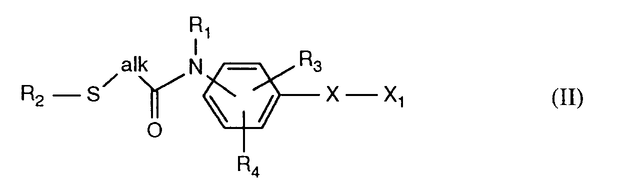 worin X₁ Carboxy oder reaktionsfähiges funktionell abgewandeltes Carboxy bedeutet, oder ein Salz davon, mit einer Verbindung der Formel III, 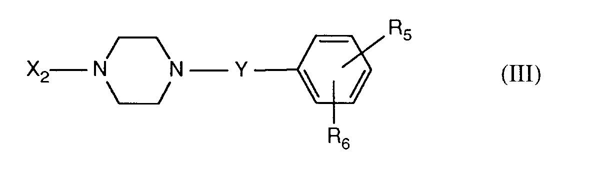 worin X₂ Wasserstoff oder eine Aminoschutzgruppe bedeutet, umsetzt, oder
b) eine Verbindung der Formel IV, 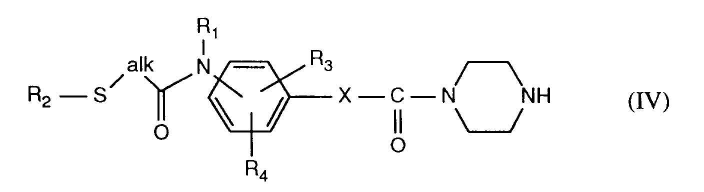 oder ein Salz davon, mit einer Verbindung der Formel V, 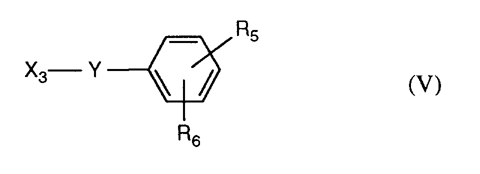 worin X₃ Hydroxy oder reaktionsfähiges verestertes Hydroxy bedeutet, umsetzt, oder
c) eine Verbindung der Formel VI, 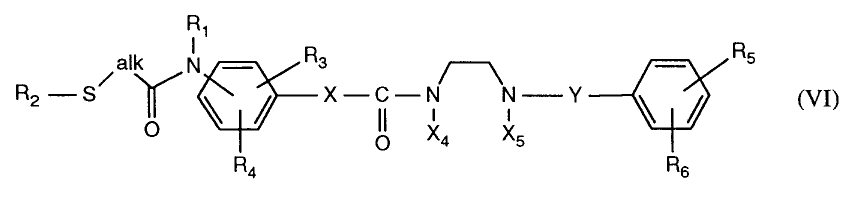 worin einer der Reste X₄ und X₅ Wasserstoff und der andere eine Gruppe der Formel -CH₂-CH₂-X₃ (VIa) bedeutet und X₃ für Hydroxy oder reaktionsfähiges verestertes Hydroxy steht, cyclisiert, oder
d) eine Verbindung der Formel VII, 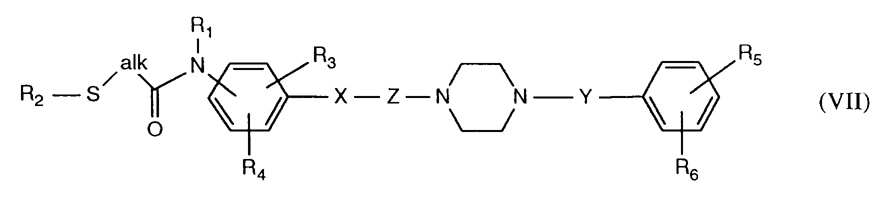 worin Z eine zu Carbonyl oxidierbare Gruppe bedeutet, oxidiert, oder
e) eine Verbindung der Formel VIII, 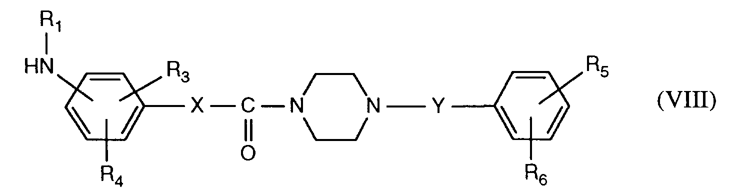 durch Umsetzung mit einer Verbindung der Formel IX, 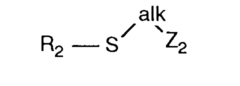 worin Z₂ Carboxy oder ein reaktionsfähiges Carboxyderivat bedeutet, acyliert, oder
f) eine Verbindung der Formel X, 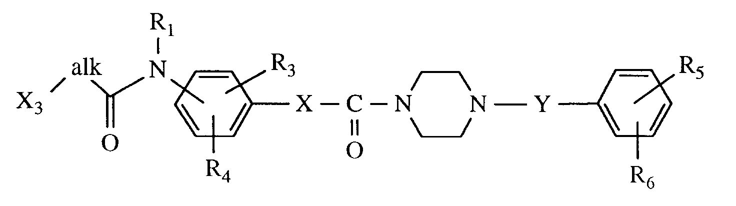 worin X₃ Hydroxy oder reaktionsfähiges verestertes Hydroxy bedeutet, mit einer Verbindung der Formel R₂-SH umsetzt;
und jeweils, wenn gewünscht, eine verfahrensgemäss oder auf andere Weise erhältliche Verbindung der Formel I in eine andere Verbindung der Formel I überführt, ein verfahrensgemäss erhältliches Isomerengemisch in die Komponenten auftrennt, eine verfahrensgemäss erhältliche freie Verbindung der Formel I in ein Salz überführt und/oder ein verfahrensgemäss erhältliches Salz in die freie Verbindung der Formel I oder in ein anderes Salz überführt.

Die vor- und nachstehend beschriebenen Umsetzungen werden in an sich bekannter Weise durchgeführt, z.B. in Ab- oder üblicher Weise in Anwesenheit eines geeigneten Lösungs- oder Verdünnungsmittels oder eines Gemisches derselben, wobei man je nach Bedarf unter Kühlen, bei Raumtemperatur oder unter Erwärmen, z.B. in einem Temperaturbereich von etwa -78° bis zur Siedetemperatur des Reaktionsmediums, vorzugsweise von etwa -10° bis etwa 150°C, und, falls erforderlich, in einem geschlossenen Gefäss, unter Druck, in einer Inertgasatmosphäre und/oder unter wasserfreien Bedingungen arbeitet.

In den Ausgangsmaterialien kann das basische Zentrum z.B. in Form von Säureadditionssalzen, beispielsweise mit den vorstehend im Zusammenhang mit Salzen von Verbindungen der Formel I aufgeführten Säuren, vorliegen, während Ausgangsverbindungen der Formel II, worin X₁ Carboxy bedeutet, Salze mit Basen bilden können. Geeignete Salze mit Basen sind beispielsweise entsprechende Alkalimetall- oder Erdalkalimetallsalze, z.B. Natrium-, Kalium- oder Magnesiumsalze, pharmazeutisch verwendbare Übergangsmetallsalze, wie Zink- oder Kupfersalze, oder Salze mit Ammoniak oder organischen Aminen, wie cyclische Amine, wie Mono-, Di- bzw. Trihydroxy-C₁-C₇-alkylamine, Hydroxy-C₁-C₇-alkyl-C₁-C₇-alkyl-amine oder wie Polyhydroxy-C₄-C₇-alkylamine. Cyclische Amine sind z.B. Morpholin, Thiomorpholin, Piperidin oder Pyrrolidin. Als Mono-C₁-C₇-alkylamine kommen beispielsweise Äthyl- oder tert.- Butylamin, als Di-C₁-C₇-alkylamine beispielsweise Diethyl- oder Diisopropylamin, als Tri-C₁-C₇-alkylamine beispielsweise Trimethyl- oder Triethylamin in Betracht. Entsprechende Hydroxy-C₁-C₇-alkylamine sind z.B. Mono-, Di- bzw. Triethanolamine, und Hydroxy-C₁-C₇-alkyl-C₁-C₇-alkylamine sind z.B. N,N-Dimethylamino- oder N,N-Diethylaminoethanol, ferner Glucosamin als Polyhydroxy-C₆-alkylamin.

Reaktionsfähiges funktionell abgewandeltes Carboxy X₁ bedeutet beispielsweise verestertes, in erster Linie reaktionsfähiges verestertes, Carboxy, anhydridisiertes Carboxy oder amidiertes Carboxy.

Verestertes Carboxy ist beispielsweise gegebenenfalls substituiertes C₁-C₇-Alkoxy-carbonyl, wie Äthoxycarbonyl, vorzugsweise jedoch reaktionsfähiges verestertes Carboxy, beispielsweise gegebenenfalls, z.B. durch C₁-C₇-Alkoxy oder gegebenenfalls substituiertes Carbamoyl, zusätzlich aktiviertes Vinyloxycarbonyl, wie 1-C₁-C₇-Alkoxy-, z.B. 1-Äthoxyvinyloxycarbonyl, oder 2-(N-C₁-C₇-Alkyl-carbamoyl)-, z.B. 2-(N-Äthylcarbamoyl)-vinyloxycarbonyl, sowie gegebenenfalls, z.B. durch Nitro, Halogen, C₁-C₇-Alkansulfonyl oder Phenylazo, substituiertes Phenoxy- bzw. Thiophenoxycarbonyl, wie 4-Nitro-, 2,4,5-Trichlor-, Pentachlor-, 4-Methansulfonyl-, 4-Phenylazo-phenoxycarbonyl, Thiophenoxy- oder 4-Nitrothiophenoxycarbonyl, und ebenso aktiviertes, z.B. durch Cyano oder ferner gegebenenfalls verestertes Carboxy substituiertes, Methoxycarbonyl, insbesondere Cyanomethoxycarbonyl. Reaktionsfähiges verestertes Carboxy kann ebenso 1,1- oder 1,3-disubstituiertes 2-Isoureidocarbonyl, wie 1,1-Diniederalkyl-, 1,1-Diaryl- oder 1,1-Diaryl-C₁-C₇-alkyl-2-isoureidocarbonyl, z.B. 1,1-Diethyl-, 1,1-Diphenyl- oder 1,1-Dibenzyl-2-isoureidocarbonyl, oder 1,3-Dicycloalkyl-, z.B. 1,3-Dicyclohexyl-2-isoureido-carbonyl, oder N-C₂-C₇-Alkylenaminooxycarbonyl, wie N-Piperidinyl-oxycarbonyl, sowie N-Imido-oxycarbonyl, z.B. N-Succinimido-oxy- oder N-Phthalimido-oxycarbonyl, sein.

Unter anhydridisiertem Carboxy ist beispielsweise gegebenenfalls verzweigtes C₁-C₇-Alkoxycarbonyloxycarbonyl, wie Äthoxy- oder Isobutyloxycarbonyloxycarbonyl, Halogencarbonyl, wie Chlorcarbonyl, Azidocarbonyl, Halogenphosphoryloxycarbonyl, wie Dichlorphosphoryloxycarbonyl, oder gegebenenfalls, z.B. durch Halogen oder Aryl, substituiertes C₁-C₇-Alkanoyloxycarbonyl, wie Pivaloyloxy-, Trifluoracetyloxy- oder Phenylacetoxycarbonyl, zu verstehen.

Reaktionsfähiges amidiertes Carboxy ist beispielsweise gegebenenfalls, z.B. durch C₁-C₇-Alkyl, substituiertes 1-Imidazolyl- oder 1-Pyrazolylcarbonyl, wie 3,5-Dimethyl-pyrazolylcarbonyl.

Eine Aminoschutzgruppe X₂ ist beispielsweise Acyl, wie C₁-C₇-Alkanoyl, z.B. Formyl oder Acetyl, Halogencarbonyl, wie Chlorcarbonyl, ferner gegebenenfalls substituiertes Aryl- oder Heteroarylsulfonyl, wie 2-Pyridyl- oder 2-Nitrophenylsulfonyl.

Im Rahmen der vor- und nachstehenden Verfahrensbeschreibung bedeutet reaktionsfähiges verestertes Hydroxy, z.B. X₃, sofern nicht abweichend definiert, insbesondere mit einer starken anorganischen Säure oder organischen Sulfonsäure verestertes Hydroxy, beispielsweise Halogen, wie Chlor, Brom oder Iod, Sulfonyloxy, wie Hydroxysulfonyloxy, Halogensulfonyloxy, z.B. Fluorsulfonyloxy, gegebenenfalls, z.B. durch Halogen, substituiertes C₁-C₇-Alkansulfonyloxy, z.B. Methan- oder Trifluormethansulfonyloxy, C₃-C₇-Cycloalkansulfonyloxy, z.B. Cyclohexansulfonyloxy, oder gegebenenfalls, z.B. durch C₁-C₇-Alkyl oder Halogen, substituiertes Benzolsulfonyloxy, z.B. p-Bromphenyl- oder p-Toluolsulfonyloxy.

Werden bei den vor- und nachstehend beschriebenen Umsetzungen beispielsweise Basen verwendet, so kommen, sofern nicht abweichend aufgeführt, beispielsweise Alkalimetallhydroxide, -hydride, -amide, -alkanolate, -carbonate, -triphenylmethylide, -di-C₁-C₇-alkylamide, -amino-C₁-C₇-alkylamide oder -C₁-C₇-alkylsilylamide, Naphthylamine, C₁-C₇-Alkylamine, basische Heterocyclen, Ammoniumhydroxide sowie carbocyclische Amine in Frage. Beispielhaft seien Lithiumhydroxid, Natriumhydroxid, -hydrid, -amid, -ethylat, Kaliumtertiärbutanolat, -carbonat, Lithiumtriphenylmethylid, -diisopropylamid, Kalium-3-(aminopropyl)amid, -bis-(trimethylsilyl)-amid, Dimethylaminonaphthalin, Di- oder Triethylamin, Pyridin, Benzyl-trimethylammoniumhydroxid, 1,5-Diaza-bicyclo[4.3.0]non-5-en (DBN) sowie 1,8-Diaza-bicyclo[5.4.0]undec-7-en (DBU) genannt.

Variante a): Die verfahrensgemässe N-Acylierung wird in an sich bekannter Weise durchgeführt, erforderlichenfalls in Gegenwart eines, insbesondere basischen, Kondensationsmittels. Als Basen kommen beispielsweise Vertreter der vorstehend aufgeführten Basen in Frage. Häufig ist auch die Basizität der Verbindung der Formel III ausreichend.

Falls X₁ Carboxy bedeutet, werden z.B. primär die entsprechenden Ammoniumsalze gebildet, die durch Erwärmen oder Behandeln mit geeigneten Dehydratisierungsmitteln (als Kondensationsmittel), wie Carbodiimiden, z.B. N,N'-Diniederalkyl- oder N,N'-Dicycloalkylcarbodiimid, wie N,N'-Diethyl-, N,N'-Diisopropyl- oder N,N'-Dicyclohexyl-carbodiimid, vorteilhaft unter Zusatz von N-Hydroxysuccinimid oder gegebenenfalls, z.B. durch Halogen, Niederalkoxy oder Niederalkyl, substituiertem 1-Hydroxy-benzotriazol oder N-Hydroxy-5-norbornen-2,3-dicarboxamid, sowie N,N-Carbonyldiimidazol, dehydratisiert werden können. Mit Carbodiimiden können intermediär z.B. auch die entsprechenden 1-Isoureidocarbonyl-Verbindungen gebildet werden. Als wasserbindende Kondensationsmittel können weiterhin N-Äthoxycarbonyl2-ethoxy-1,2-dihydrochinolin, Phosphorylcyanamide bzw. -azide, wie Diethylphosphorylcyanamid oder Diphenylphosphorylazid, Triphenylphosphin-disulfid oder 1-Niederalkyl-2-halogeno-piperidinium-halogenide, wie 1-Methyl-2-chlor- pyridinium-iodid, eingesetzt werden.

Die in dieser Verfahrensvariante verwendeten Ausgangsstoffe sind z.T. bekannt bzw. können nach an sich bekannten Verfahren hergestellt werden.

Zur Herstellung von Verbindungen der Formel II, worin X₁ gegebenenfalls substituiertes C₁-C₇-Alkoxycarbonyl bedeutet, kann man üblicherweise von der freien Säure (X₁ = Carboxy) oder einem Säureanhydrid (X₁ bedeutet z.B. Halogencarbonyl) ausgehen und diese beispielsweise mit dem entsprechenden, erforderlichenfalls in reaktionsfähiger Form vorliegenden, Alkohol, z.B. einem C₁-C₇-Alkylhalogenid, umsetzen. Die Herstellung von Verbindungen der Formel II, worin X₁ gegebenenfalls zusätzlich aktiviertes Vinyloxycarbonyl bedeutet, kann z.B. durch Umesterung eines C₁-C₇-Alkylesters mit Vinylacetat (Methode des aktivierten Vinylesters), durch Umsetzung der freien Säure von Verbindungen der Formel II mit Niederalkoxyacetylen (z.B. Äthoxyacetylen-Methode) oder, analog der Woodward-Methode, mit einem 1,2-Oxazoliumsalz erfolgen. Gegebenenfalls substituiertes Phenoxy- bzw. Thiophenoxycarbonyl aufweisende Verbindungen der Formel II können beispielsweise ausgehend von der freien Säure nach der Carbodiimid-Methode durch Umsetzen mit dem entsprechenden (Thio-)Phenol durchgeführt werden. Ebenfalls ausgehend von der freien Säure der Formel II können Verbindungen der Formel II, worin X₁ aktiviertes Methoxycarbonyl bzw. 1,1- oder 1,3-disubstituiertes 2-Isoureidocarbonyl darstellt, z.B. durch Umsetzung mit einem Halogen-, wie Chloracetonitril (Cyanmethylester-Methode) bzw. mit einem Carbodiimid oder Cyanamid (Carbodiimid- oder Cyanamid-Methode) erhalten werden. Die Herstellung von N-C₂-C₇-Alkylenamino-oxycarbonyl- bzw. N-Imido-oxycarbonyl-Verbindungen der Formel II kann beispielsweise bei Verwendung der freien Säure der Formel II aus entsprechenden N-Hydroxyverbindungen mit Hilfe von Carbodiimiden nach der Methode der aktivierten N-Hydroxyester erfolgen. Zur Herstellung von Verbindungen der Formel II, worin X₁ gegebenenfalls verzweigtes C₁-C₇-Alkoxycarbonyloxycarbonyl, Halogenphosphoryloxycarbonyl bzw. gegebenenfalls substituiertes C₁-C₇-Alkanoyloxycarbonyl bedeutet, kann beispielsweise von freier Säure der Formel II ausgegangen werden, die z.B. mit einem entsprechenden Halogenid, wie gegebenenfalls substituiertes C₁-C₇-Alkylkohlensäure-halogenid (Methode der gemischten O-Kohlensäureanhydride), Phosphoroxyhalogenid (z.B. Phosphoroxychlorid-Methode) oder gegebenenfalls substituiertes C₁-C₇-Alkanoyl-halogenid (Methode der gemischten Carbonsäurehalogenide) behandelt werden kann. Azidocarbonylverbindungen der Formel II sind beispielsweise durch Behandeln entsprechender Hydrazide mit salpetriger Säure zugänglich (Azid-Methode). Zur Herstellung von Verbindungen der Formel II, worin X₁ gegebenenfalls substituiertes 1-Imidazolyl-carbonyl bzw. 1-Pyrazolyl-carbonyl bedeutet, setzt man die freie Säure der Formel II z.B. mit Di-(1-imidazolyl)-carbonyl (Imidazolid-Methode) bzw. das betreffende Hydrazid z.B. mit einem entsprechenden 1,3-Diketon (Pyrazolid-Methode) um.

Variante b): Der Rest X₃ bedeutet insbesondere reaktionsfähiges verestertes Hydroxy, z.B. Halogen, wie Chlor.

Die verfahrensgemässe N-Alkylierung wird in an sich bekannter Weise durchgeführt, erforderlichenfalls in Gegenwart einer, z.B. der vorstehend aufgeführten, Basen.

Die in dieser Verfahrensvariante verwendeten Ausgangsstoffe sind z.T. bekannt oder können in an sich bekannter Weise hergestellt werden.

So kann beispielsweise das Ausgangsmaterial der Formel IV hergestellt werden, indem man eine Verbindung der Formel II oder ein Salz davon, worin X₁ Carboxy oder reaktionsfähiges funktionell abgewandeltes Carboxy bedeutet, mit einer Verbindung der Formell IVa 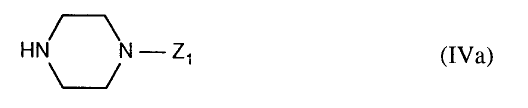
oder einem Salz davon, worin Z₁ Wasserstoff oder eine Aminoschutzgruppe, wie Benzyl, bedeutet, in der in Variante a) beschriebenen Weise umsetzt und gegebenenfalls die Aminoschutzgruppe, z.B. Benzyl durch übliche Hydrogenolyse, abspaltet.

Variante c): Die verfahrensgemässe Cyclisierung (intramolekulare N-Alkylierung) wird in an sich bekannter Weise, erforderlichenfalls in Gegenwart eines, insbesondere basischen, Kondensationsmittels durchgeführt. Als Basen werden z.B. vorstehend aufgeführten verwendet.

X₃ bedeutet dabei insbesondere reaktionsfähiges verestertes Hydroxy, bevorzugt Halogen, wie Chlor.

Das Ausgangsmaterial kann in an sich bekannter Weise hergestellt werden. Beispielsweise geht man von einer Verbindung der Formel II oder einem Salz davon aus, worin X₁ Carboxy oder reaktionsfähiges funktionell abgewandeltes Carboxy bedeutet, und setzt diese zunächst mit einer Verbindung der Formel 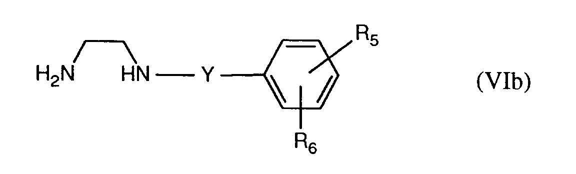
in Analogie zu Variante a) um. Im nächsten Reaktionsschritt wird die erhaltene Verbindung mit einer Verbindung der Formel X₃-CH₂-CH₂-X₃ (VIc) unter N-alkylierenden Bedingungen gemäss Variante b) umgesetzt.

Variante d): Eine zu -CO- oxidierbare Gruppe Z steht in erster Linie für -CH₂-. Die Oxidation entsprechender Verbindungen der Formel VII erfolgt mit Hilfe eines geeigneten Oxidationsmittels, wobei bevorzugt gegebenenfalls, z.B. durch einen Phenylrest, substituierte Tetra-C₁-C₄-alkylammoniumpermanganate, in erster Linie Benzyl-triethylammoniumpermanganat, verwendet wird.

Das Ausgangsmaterial der Formel VII wird in an sich bekannter Weise hergestellt. Beispielsweise geht man von einer Verbindung der Formel III aus, worin X₂ Wasserstoff bedeutet, und setzt diese unter den in Variante b) beschriebenen N-alkylierenden Bedingungen mit einer Verbindung der Formel VIIa 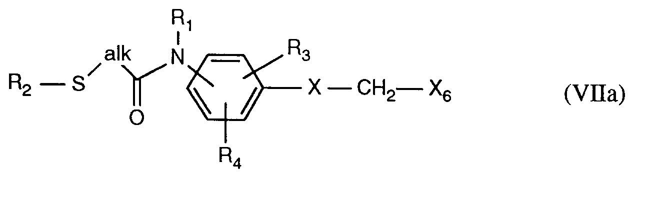
um, wobei X₆ Hydroxy oder in erster Linie reaktionsfähiges verestertes Hydroxy, insbesondere Halogen, wie Chlor oder Brom, bedeutet.

Variante e): Ein reaktionsfähiges Carboxyderivat ist z.B. ein Säurehalogenid oder -anhydrid, oder eines der oben aufgeführten reaktionsfähigen veresterten Carboxyderivate.

Die Einführung des Acylrestes gemäss Variante e) (N-Acylierung) erfolgt in üblicher Weise [siehe auch Variante a)], erforderlichenfalls in Gegenwart eines, insbesondere basischen, Kondensationsmittels. Als Basen kommen beispielsweise Vertreter der oben aufgeführten Basen in Frage.

Variante f): Die Variante f) betrifft die an sich bekannte S-Alkylierung von Mercaptanen. Bei der Umsetzung wird, falls erforderlich, eine Base zugegeben, z.B. wässrige Ammoniak-Lösung oder eine andere der oben aufgeführten Basen.

Wenn bei der Umsetzung gemäss f) die Verbindung R₂-SH z.B. für L-Glutathion oder ein Derivat davon steht, kann anstelle einer Base auch ein entsprechendes Enzym, z.B. Glutathion-S-transferase, hinzugegeben werden.

Eine verfahrensgemäss oder anderweitig erhältliche erfindungsgemässe Verbindung kann in an sich bekannter Weise in eine andere erfindungsgemässe Verbindung überführt werden, beispielsweise durch an sich bekannte Umsetzungen wie die Verseifung von Estern zu Carbonsäuren, die Veresterung von Carbonsäuren, oder die N-Alkylierung oder N-Acylierung von Aminogruppen.

Erhaltene Salze können in an sich bekannter Weise in die freien Verbindungen umgewandelt werden, z.B. durch Behandeln mit einer Base, wie einem Alkalimetallhydroxid, einem Metallcarbonat oder -hydrogencarbonat, oder Ammoniak, oder einer anderen eingangs genannten salzbildenden Base bzw. mit einer Säure, wie einer Mineralsäure, z.B. mit Chlorwasserstoff, oder einer anderen eingangs genannten salzbildenden Säure.

Erhaltene Salze können in an sich bekannter Weise in andere Salze überführt werden, Säureadditionssalze z.B. durch Behandeln mit einem geeigneten Metallsalz, wie einem Natrium-, Barium- oder Silbersalz, einer anderen Säure in einem geeigneten Lösungsmittel, in welchem ein sich bildendes anorganisches Salz unlöslich ist und damit aus dem Reaktionsgleichgewicht ausscheidet, und Basesalze durch Freisetzung der freien Säure und erneute Versalzung.

Die Verbindungen der Formel I, einschliesslich ihrer Salze, können auch in Form von Hydraten erhalten werden oder das zur Kristallisation verwendete Lösungsmittel einschliessen.

Infolge der engen Beziehung zwischen den neuen Verbindungen in freier Form und in Form ihrer Salze sind vorstehend und nachfolgend unter den freien Verbindungen und ihren Salzen sinn- und zweckgemäss gegebenenfalls auch die entsprechenden Salze bzw. freien Verbindungen zu verstehen.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Salzes verwendet oder insbesondere unter den Reaktionsbedingungen bildet.

Die neuen Ausgangsstoffe, die speziell für die Herstellung der erfindungsgemässen Verbindungen entwickelt wurden, insbesondere die zu den eingangs als bevorzugt gekennzeicheten Verbindungen der Formel I führende Ausgangsstoffauswahl, die Verfahren zu ihrer Herstellung und ihre Verwendung als Zwischenprodukte bilden ebenfalls einen Gegenstand der Erfindung.

Die neuen Verbindungen der Formel I können z.B. in Form pharmazeutischer Präparate Verwendung finden, welche eine therapeutisch wirksame Menge der Aktivsubstanz, gegebenenfalls zusammen mit anorganischen oder organischen, festen oder flüssigen, pharmazeutisch verwendbaren Trägerstoffen enthalten, die sich zur enteralen, z.B. oralen, oder parenteralen Verabreichung eignen. So verwendet man Tabletten oder Gelatinekapseln, welche den Wirkstoff zusammen mit Verdünnungsmitteln, z.B. Lactose, Dextrose, Saccharose, Mannit, Sorbit, Cellulose und/oder Schmiermitteln, z.B. Kieselerde, Talk, Stearinsäure oder Salzen davon, wie Magnesium- oder Calciumstearat, und/oder Polyethylenglykol, aufweisen. Tabletten können ebenfalls Bindemittel, z.B. Magnesiumaluminiumsilikat, Stärken, wie Mais-, Weizen-, Reis- oder Pfeilwurzstärke, Gelatine, Traganth, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, z.B. Natriumalginat, und/oder Brausemischungen, oder Absorptionsmittel, Farbstoffe, Geschmacksstoffe und Süssmittel aufweisen. Ferner kann man die neuen Verbindungen der Formel I z.B. in Form von parenteral verabreichbaren Präparaten oder von Infusionslösungen verwenden. Solche Lösungen sind vorzugsweise isotonische wässrige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservierungs-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wirksame Stoffe enthalten können, werden in an sich bekannter Weise, z.B. mittels konventioneller Misch-, Granulier-, Dragier-, Lösungs- oder Lyophilisierungsverfahren hergestellt und enthalten von etwa 0,1% bis 100%, insbesondere von etwa 1% bis etwa 50%, Lyophilisate bis etwa 100% des Aktivstoffes.

Die Erfindung betrifft ebenfalls die Verwendung der Verbindungen der Formel I, vorzugsweise in Form von pharmazeutischen Präparaten. Die Dosierung kann von verschiedenen Faktoren, wie Applikationsweise, Spezies, Alter und/oder individuellem Zustand abhängen. Die täglich zu verabreichenden Dosen liegen bei oraler Applikation zwischen etwa 0,25 und etwa 10 mg/kg und für Warmblüter mit einem Körpergewicht von etwa 70 kg vorzugsweise zwischen etwa 20 mg und etwa 500 mg.

Die nachfolgenden Beispiele dienen zur Illustration der Erfindung; Temperaturen sind in Celsiusgraden, Drucke in mbar angegeben. Folgende Abkürzungen werden verwendet: RT = Raumtemperatur, AT = Aussentemperatur, IT = Innentemperatur, Ether = Diethylether, Essigester = Ethylacetat = Essigsäureethylester, (BOC)₂O = Di-tert.-Butyl-dicarbonat.

### Beispiel 1: (-)-1-{4-[N-(2-Isopropyloxyethyl)-N-([2R]-2-methoxycarbonyl-2-aminoethyl-mercapto-acetyl)-amino]-benzoyl}-4-[2-(4-chlorphenyl)-ethyl]-piperazindihydrochlorid-trihydrat

2 g 1-{4-[N-(2-Isopropyloxyethyl)-N-chloracetyl-amino]-benzoyl}-4-[2-(4-chlorphenyl)--ethyl]-piperazin-hydrochlorid, Smp. 146-147°, (s. EP-A-524 146, Beispiele 3 und 2) werden in 50 ml Wasser suspendiert und vorgelegt. Man gibt 3.16 g L-Cysteinmethylester-hydrochlorid (Fa. Fluka) und anschliessend 20 ml Ether zu. Unter Rühren werden 2 ml konz. wässrige Ammoniak-Lösung hinzugegeben. Es ensteht eine Emulsion mit einem pH von etwa 10. Innerhalb von kurzer Zeit bilden sich klare Schichten. Nach 2h heftigem Rühren wird die zweiphasige Reaktionsmischung mit Ether verdünnt; die organische Phase wird abgetrennt und mit Wasser gewaschen, getrocknet und eingedampft. Das ölige Material wird mit etherischer Salzsäure versetzt und aus Dichlormethan-Ether kristallisiert. Man erhält so die Titelverbindung vom Smp. 125-127°; α_{D}²⁵ = -14.8° (c = 4.94%, Methanol).

Durch geeignete Hydrolyse des Esters des Beispiels 1 in üblicher Weise, z.B. durch Umsetzung im sauren Medium, erhält man die folgenden Säure:
(a) 1-{4-[N-(2-Isopropyloxyethyl)-N-([2R]-2-carboxy-2-aminoethylmercapto-acetyl)-amino]-benzoyl}-4-[2-(4-chlorphenyl)-ethyl]-piperazin.
   Setzt man anstelle des L-Cystein-methylesters einen entsprechenden anderen L-Cystein-ester gemäss dem Verfahren des Beispiels 1 um, so erhält man die folgenden Ester:
(b) 1-{4-[N-(2-Isopropyloxyethyl)-N-([2R]-2-ethoxycarbonyl-2-aminoethylmercapto-acetyl)-amino]-benzoyl}-4-[2-(4-chlorphenyl)-ethyl]-piperazin. Das Dihydrochloridmonohydrat davon hat einen Smp. von 123-126° und ein α_{D}²⁵= - 16.5° (c = 0.606%, Methanol).
(c) 1-{4-[N-(2-Isopropyloxyethyl)-N-([2R]-2-n-propyloxycarbonyl-2-aminoethylmercapto-acetyl)-amino]-benzoyl}-4-[2-(4-chlorphenyl)-ethyl]-piperazin
(d) 1-{4-[N-(2-Isopropyloxyethyl)-N-([2R]-2-isopropyloxycarbonyl-2-aminoethylmercapto-acetyl)-amino]-benzoyl}-4-[2-(4-chlorphenyl)-ethyl]-piperazin
(e) 1-{4-[N-(2-Isopropyloxyethyl)-N-([2R]-2-n-butyloxycarbonyl-2-aminoethylmercapto-acetyl)-amino]-benzoyl}-4-[2-(4-chlorphenyl)-ethyl]-piperazin
(f) 1-{4-[N-(2-Isopropyloxyethyl)-N-([2R]-2-sek-butyloxycarbonyl-2-aminoethylmercapto-acetyl)-amino]-benzoyl}-4-[2-(4-chlorphenyl)-ethyl]-piperazin
(g) 1-{4-[N-(2-Isopropyloxyethyl)-N-([2R]-2-isobutyloxycarbonyl-2-aminoethylmercapto-acetyl)-amino]-benzoyl}-4-[2-(4-chlorphenyl)-ethyl]-piperazin
(h) 1-{4-[N-(2-Isopropyloxyethyl)-N-([2R]-2-tert-butyloxycarbonyl-2-aminoethylmercapto-acetyl)-amino]-benzoyl}-4-[2-(4-chlorphenyl)-ethyl]-piperazin.

Die Ester 1(b) bis 1(h) können weiterhin auch durch Veresterung der Säure des Beispiels 1(a) in geeigneter Weise, z.B. durch Umsetzung mit dem entsprechenden Alkanol, erhalten werden.

Beispiel 2: Analog zu Beispiel 1 wird das 1-{4-[N-(2-Isopropyloxyethyl)-N-chloracetyl-amino]-benzoyl}-4-[2-(4-chlorphenyl)-ethyl]-piperazin-hydrochlorid mit N-Acetyl-3-mercapto-D-valin (= N-Acetyl-D-penicillamin) umgesetzt, wobei man das 1-{4-[N-(2-Isopropyloxyethyl)-N-([2R]-2-carboxy-2-N'-acetylamino-1,1-dimethyl-ethylmercapto-acetyl)-amino]-benzoyl}-4-[2-(4-chlorphenyl)-ethyl]-piperazin erhält.

Setzt man anstelle von N-Acetyl-3-mercapto-D-valin einen entsprechenden Alkylester gemäss dem Verfahren des Beispiels 2 um, so erhält man die folgenden Ester:
(a) 1-{4-[N-(2-Isopropyloxyethyl)-N-([2R]-2-methoxycarbonyl-2-N'-acetylamino-1,1-dimethyl-ethylmercapto-acetyl)-amino]-benzoyl}-4-[2-(4-chlorphenyl)-ethyl]-piperazin
(b) 1-{4-[N-(2-Isopropyloxyethyl)-N-([2R]-2-ethoxycarbonyl-2-N'-acetylamino-1,1-dimethyl-ethylmercapto-acetyl)-amino]-benzoyl}-4-[2-(4-chlorphenyl)-ethyl]-piperazin.

Die Ester 2(a) und 2(b) können weiterhin auch durch Veresterung der Säure des Beispiels 2 in geeigneter Weise, z.B. durch Umsetzung mit dem entsprechenden Alkanol, erhalten werden.

Beispiel 3: Analog zu Beispiel 1 wird das 1-{4-[N-(2-Isopropyloxyethyl)-N-chloracetylamino]-benzoyl}-4-[2-(4-chlorphenyl)-ethyl]-piperazin-hydrochlorid mit 3-Mercapto-DL-valin (= DL-Penicillamin) umgesetzt, wobei man (±)-1-{4-[N-(2-Isopropyloxyethyl)-N-(2-carboxy-2-amino-1,1-dimethyl-ethylmercapto-acetyl)-amino]-benzoyl}-4-[2-(4-chlorphenyl)-ethyl]-piperazin erhält.

Setzt man anstelle von 3-Mercapto-DL-valin einen entsprechenden Alkylester gemäss dem Verfahren des Beispiels 3 um, so erhält man die folgenden Ester:
(a)(±)-1-{4-[N-(2-Isopropyloxyethyl)-N-(2-methoxycarbonyl-2-amino-1,1-dimethyl-ethylmercapto-acetyl)-amino]-benzoyl}-4-[2-(4-chlorphenyl)-ethyl]-piperazin
(b)(±)-1-{4-[N-(2-Isopropyloxyethyl)-N(2-ethoxycarbonyl-2-amino-1,1-dimethyl-ethylmercapto-acetyl)-amino]-benzoyl}-4-[2-(4-chlorphenyl)-ethyl]-piperazin.

Die Ester 3(a) und 3(b) können weiterhin auch durch Veresterung der Säure des Beispiels 3 in geeigneter Weise, z.B. durch Umsetzung mit dem entsprechenden Alkanol, erhalten werden.

Beispiel 4: Analog zu Beispiel 1 wird das 1-{4-[N-(2-Isopropyloxyethyl)-N-chloracetyl-amino]-benzoyl}-4-[2-(4-chlorphenyl)-ethyl]-piperazin-hydrochlorid mit DL-2-Mercapto-bernsteinsäure (= DL-Thioäpfelsäure) umgesetzt, wobei man (±)-1-{4-[N-(2-Isopropyloxyethyl)-N-(1,2-dicarboxyethylmercapto-acetyl)-amino]-benzoyl}-4-[2-(4-chlorphenyl)-ethyl]-piperazin erhält.

Setzt man anstelle von DL-2-Mercapto-bernsteinsäure einen entsprechenden Dialkylester gemäss dem Verfahren des Beispiels 4 um, so erhält man die folgenden Ester:
(a)(±)-1-{4-[N-(2-Isopropyloxyethyl)-N-(1,2-di-methoxycarbonyl-ethylmercapto-acetyl)-amino]-benzoyl}-4-[2-(4-chlorphenyl)-ethyl]-piperazin
(b)(±)-1-{4-[N-(2-Isopropyloxyethyl)-N-(1,2-di-ethoxycarbonyl-ethylmercapto-acetyl)-amino]-benzoyl}-4-[2-(4-chlorphenyl)-ethyl]-piperazin.

Die Ester 4(a) und 4(b) können weiterhin auch durch Veresterung der Dicarbonsäure des Beispiels 4 in geeigneter Weise, z.B. durch Umsetzung mit dem entsprechenden Alkanol, erhalten werden.

Beispiel 5: Analog zu Beispiel 1 werden 2.0 g von 1-{4-[N-(2-Isopropyloxyethyl)-N-chloracetyl-amino]-benzoyl}-4-[2-(4-chlorphenyl)-ethyl]-piperazin - aus dem entsprechenden Hydrochlorid durch Behandlung mit einem Gemisch aus wässriger Ammoniaklösung/Methylenchlorid freigesetzt - mit 0.571 g N-Acetyl-L-cystein umgesetzt, wobei man das 1-{4-[N-(2-Isopropyloxyethyl)-N-([2R]-2-carboxy-2-N'-acetylamino-ethylmercapto-acetyl)-amino]-benzoyl}-4-[2-(4-chlorphenyl)-ethyl]-piperazin erhält. Das Hydrochlorid-hexahydrat davon hat einen Smp. von 132-135° und ein α_{D}²⁵=-12.5° (c = 1.095%, Methanol).

Setzt man anstelle von N-Acetyl-L-cystein einen entsprechenden Alkylester gemäss dem Verfahren des Beispiels 5 um, so erhält man die folgenden Ester:
(a) 1-{4-[N-(2-Isopropyloxyethyl)-N-([2R]-2-methoxycarbonyl-2-N'-acetylamino-ethylmercapto-acetyl)-amino]-benzoyl}-4-[2-(4-chlorphenyl)-ethyl]-piperazin
(b) 1-{4-[N-(2-Isopropyloxyethyl)-N([2R]-2-ethoxycarbonyl-2-N'-acetylamino-ethylmercapto-acetyl)-amino]-benzoyl}-4-[2-(4-chlorphenyl)-ethyl]-piperazin
(c) 1-{4-[N-(2-Isopropyloxyethyl)-N-([2R]-2-n-propyloxycarbonyl-2-N'-acetylaminoethylmercapto-acetyl)-amino]-benzoyl}-4-[2-(4-chlorphenyl)-ethyl]-piperazin
(d) 1-{4-[N-(2-Isopropyloxyethyl)-N-([2R]-2-isopropyloxycarbonyl-2-N'-acetylaminoethylmercapto-acetyl)-amino]-benzoyl}-4-[2-(4-chlorphenyl)-ethyl]-piperazin
(e) 1-{4-[N-(2-Isopropyloxyethyl)-N-([2R]-2-n-butyloxycarbonyl-2-N'-acetylamino-ethylmercapto-acetyl)-amino]-benzoyl}-4-[2-(4-chlorphenyl)-ethyl]-piperazin
(f) 1-{4-[N-(2-Isopropyloxyethyl)-N-([2R]-2-sek-butyloxycarbonyl-2-N'-acetylaminoethylmercapto-acetyl)-amino]-benzoyl}-4-[2-(4-chlorphenyl)-ethyl]-piperazin
(g) 1-{4-[N-(2-Isopropyloxyethyl)-N-([2R]-2-isobutyloxycarbonyl-2-N'-acetylaminoethylmercapto-acetyl)-amino]-benzoyl}-4-[2-(4-chlorphenyl)-ethyl]-piperazin
(h) 1-{4-[N-(2-Isopropyloxyethyl)-N-([2R]-2-tert-butyloxycarbonyl-2-N'-acetylaminoethylmercapto-acetyl)-amino]-benzoyl}-4-[2-(4-chlorphenyl)-ethyl]-piperazin.

Die Ester 5(a) bis 5(h) können weiterhin auch durch Veresterung der Säure des Beispiels 5 in geeigneter Weise, z.B. durch Umsetzung mit dem entsprechenden Alkanol, erhalten werden.

Beispiel 6: Analog zu Beispiel 1 wird 1.0 g 1-{4-[N-(2-Isopropyloxyethyl)-N-chloracetyl-amino]-benzoyl}-4-[2-(4-chlorphenyl)-ethyl]-piperazin - aus dem entsprechenden Hydrochlorid durch Behandlung mit einem Gemisch aus wässriger Ammoniaklösung/Methylenchlorid freigesetzt - mit 0.24 g 3-Mercapto-propionsäure-methylester umgesetzt, wobei man das 1-{4-[N-(2-Isopropyloxyethyl)-N-(2-methoxycarbonyl-ethylmercapto-acetyl)-amino]-benzoyl}-4-[2-(4-chlorphenyl)-ethyl]-piperazin erhält. Das Hydrochlorid-monohydrat davon hat einen Smp. von 91-94°.

Durch geeignete Hydrolyse des Esters des Beispiels 6 in üblicher Weise, z.B. durch Umsetzung im sauren Medium, erhält man die folgenden Säure:
(a) 1-{4-[N-(2-Isopropyloxyethyl)-N-(2-carboxy-ethylmercapto-acetyl)-amino]-benzoyl}-4-[2-(4-chlorphenyl)-ethyl]-piperazin.

Beispiel 7: Analog zu Beispiel 1 wird das 1-{4-[N-(2-Isopropyloxyethyl)-N-chloracetyl-amino]-benzoyl}-4-[2-(4-chlorphenyl)-ethyl]-piperazin-hydrochlorid mit 3-Mercapto-2-oxo-propionsäure (= Mercaptobrenztraubensäure) umgesetzt, wobei man 1-{4-[N-(2-Isopropyloxyethyl)-N-(2-carboxy-2-oxo-ethylmercapto-acetyl)-amino]-benzoyl}-4-[2-(4-chlorphenyl)-ethyl]-piperazin erhält.

Setzt man anstelle von 3-Mercapto-2-oxo-propionsäure einen entsprechenden Alkylester gemäss dem Verfahren des Beispiels 7 um, so erhält man die folgenden Ester:
(a) 1-{4-[N-(2-Isopropyloxyethyl)-N-(2-methoxycarbonyl-2-oxo-ethylmercapto-acetyl)-amino]-benzoyl}-4-(2-(4-chlorphenyl)-ethyl]-piperazin
(b) 1-{4-[N-(2-Isopropyloxyethyl)-N-(2-ethoxycarbonyl-2-oxo-ethylmercapto-acetyl)-amino]-benzoyl}-4-[2-(4-chlorphenyl)-ethyl]-piperazin.

Die Ester 7(a) und 7(b) können weiterhin auch durch Veresterung der Säure des Beispiels 7 in geeigneter Weise, z.B. durch Umsetzung mit dem entsprechenden Alkanol, erhalten werden.

Beispiel 8: 1 g 1-{4-[N-(2-Isopropyloxyethyl)-N-chloracetyl-amino]-benzoyl}-4-[2-(4-chlorphenyl)-ethyl]-piperazin-hydrochlorid, Smp. 146-147°, werden in 40 ml Wasser suspendiert und vorgelegt. Anschliessend werden 20 ml Ether zugegeben, wobei das Hydrochlorid fast vollständig gelöst wird. Man gibt nun 0.4 g L-Glutathion (reduzierte Form, Fa. Fluka) [= γ-L-Glutamyl-L-cysteinyl-glycin] zu. Dann fügt man 1 ml konz. wässrige Ammoniak-Lösung zu. Es ensteht eine milchige Emulsion mit einem pH von etwa 12. Innerhalb von kurzer Zeit bilden sich klare Schichten. Nach 14h starkem Rühren unter einem permanenten N₂-Strom wird die zweiphasige Reaktionsmischung mit Ether verdünnt; die organische Phase wird abgetrennt und mit Wasser gewaschen, getrocknet und eingedampft. Das glasige Material wird mit 10 ml Methanol verrührt und die so erhaltene Lösung mit 150 ml Aceton verdünnt. Es fällt ein klumpiges Material aus. Nach dem Abdekantieren wird das Material mit 30 ml Aceton versetzt und unter Erwärmung verrührt. Man erhält ein kristallines Material, das abgenutscht wird. Dieses entspricht dem 1-{{4-{N-(2-Isopropyloxyethyl)-N-[[[2R]-2-[N'-(carboxymethyl)carbamoyl]-2-[N''-(γ-L-glutamyl)amino]-ethylmercapto-acetyl]]-amino}-benzoyl}}-4-[2-(4-chlorphenyl)-ethyl]-piperazin vom Smp. 126-129°. Trocknet man die Substanz, löst sie in Methanol und fällt sie durch Zugabe von Ether wieder aus, so erhält man ein Produkt vom Smp. 168-173°.

Beispiel 9: Analog zu Beispiel 1 wird das 1-{4-[N-(2-Isopropyloxyethyl)-N-chloracetyl amino]-benzoyl}-4-[2-(4-chlorphenyl)-ethyl]-piperazin-hydrochlorid mit DL-Homocystein (= 2-Amino-4-mercapto-buttersäure) umgesetzt, wobei man das (±)-1-{4-[N-(2-Isopropyloxyethyl)-N-(3-carboxy-3-amino-propylmercapto-acetyl)-amino]-benzoyl}-4-[2-(4-chlorphenyl)-ethyl]-piperazin erhält.

Setzt man anstelle von DL-Homocystein einen entsprechenden Alkylester gemäss dem Verfahren des Beispiels 9 um, so erhält man die folgenden Ester:
(a) (±)-1-{4-[N-(2-Isopropyloxyethyl)-N-(3-methoxycarbonyl-3-amino-propylmercapto-acetyl)-amino]-benzoyl}-4-[2-(4-chlorphenyl)-ethyl]-piperazin
(b)(±)-1-{4-[N-(2-Isopropyloxyethyl)-N-(3-ethoxycarbonyl-3-amino-propylmercapto-acetyl)-amino]-benzoyl}-4-[2-(4-chlorphenyl)-ethyl]-piperazin.

Die Ester 9(a) und 9(b) können weiterhin auch durch Veresterung der Säure des Beispiels 9 in geeigneter Weise, z.B. durch Umsetzung mit dem entsprechenden Alkanol, erhalten werden.

Beispiel 10: Analog zu Beispiel 1 werden 1.6 g 1-{4-[N-(2-Isopropyloxyethyl)-N-chloracetyl-amino]-benzoyl}-4-[2-(4-chlorphenyl)-ethyl]-piperazin - aus dem entsprechenden Hydrochlorid durch Behandlung mit einem Gemisch aus wässriger Ammoniaklösung/Methylenchlorid freigesetzt - mit 0.55 g L-Cysteinyl-glycin umgesetzt, wobei man das 1-{{4-(N-(2-Isopropyloxyethyl)-N-[[[2R]-2-[N'-(carboxymethyl)-carbamoyl]-2-amino-ethylmercapto-acetyl]]-amino}-benzoyl}}-4-[2-(4-chlorphenyl)-ethyl]-piperazin erhält. Das Hemihydrat davon hat einen Smp. von 85-87° und ein α_{D²⁵=} -1.5° (c = 0.545%, Methanol).

Beispiel 10a: Analog zu Beispiel 1 wird das 1-{4-[N-(2-Isopropyloxyethyl)-N-chloracetyl-amino]-benzoyl}-4-[2-(4-chlorphenyl)-ethyl]-piperazin-hydrochlorid mit γ-L-Glutamyl-L-cystein umgesetzt, wobei man das 1-{{4-{N-(2-Isopropyloxyethyl)-N-[[[2R]-2-carboxy-2-[N'-(γ-L-glutamyl)amino]-ethylmercapto-acetyl]]-amino)-benzoyl}}-4-[2-(4-chlorphenyl)-ethyl]-piperazin erhält.

Beispiel 11: Analog zu Beispiel 1 wird das 1-{4-[N-(2-Isopropyloxyethyl)-N-chloracetyl-amino]-benzoyl}-4-[2-(4-chlorphenyl)-ethyl]-piperazin-hydrochlorid mit D-Cystein-methylester umgesetzt, wobei man das (+)-1-{4-[N-(2-Isopropyloxyethyl)-N-([2S]-2-methoxycarbonyl-2-aminoethylmercapto-acetyl)-amino]-benzoyl}-4-[2-(4-chlorphenyl)-ethyl]-piperazin erhält.

Beispiel 12: Analog zu Beispiel 1 werden 2.0 g 1-{4-[N-(2-Isopropyloxyethyl)-N-chloracetyl-amino]-benzoyl}-4-[2-(4-chlorphenyl)-ethyl]-piperazin - aus dem entsprechenden Hydrochlorid durch Behandlung mit einem Gemisch aus wässriger Ammoniaklösung/Methylenchlorid freigesetzt - mit 1.29 g N-Acetyl-L-Glutathion (reduzierte Form) [= γ-N-Acetyl-L-glutamyl-L-cysteinyl-glycin] umgesetzt, wobei man das 1-{{4-{N-(2-Isopropyloxyethyl)-N-[[[2R]-2-[N'-(carboxymethyl)carbamoyl]-2-[N''-(γ-N'''-acetyl-L-glutamyl)amino]-ethylmercapto-acetyl]]-amino}-benzoyl}}-4-[2-(4-chlorphenyl)-ethyl]-piperazin erhält. Das Trihydrat davon hat einen Smp. von 149-152° und ein α_{D}²⁵= -23.5° (c = 0.498%, Methanol).

Beispiel 13: Analog zu Beispiel 1 wird das 1-{4-[N-(2-Isopropyloxyethyl)-N-chloracetyl-amino]-benzoyl}-4-[2-(4-chlorphenyl)-ethyl]-piperazin-hydrochlorid mit 2-Mercapto-essigsäure-methylester umgesetzt, wobei man das 1-{4-[N-(2-Isopropyloxyethyl)-N-(methoxycarbonyl-methylmercapto-acetyl)-amino]-benzoyl}-4-[2-(4-chlorphenyl)-ethyl]-piperazin erhält.

Beispiel 14: Analog zu Beispiel 1 wird das 1-{4-[N-(2-Isopropyloxyethyl)-N-chloracetylamino]-benzoyl}-4-[2-(4-chlorphenyl)-ethyl]-piperazin-hydrochlorid mit 4-Mercapto-buttersäure umgesetzt, wobei man das 1-{4-[N-(2-Isopropyloxyethyl)-N-(3-carboxy-propylmercapto-acetyl)-amino]-benzoyl}-4-[2-(4-chlorphenyl)-ethyl]-piperazin erhält.

Beispiel 15: Analog zu Beispiel 1 wird das 1-{4-[N-(2-Isopropyloxyethyl)-N-chloracetylamino]-benzoyl}-4-[2-(4-chlorphenyl)-ethyl]-piperazin-hydrochlorid mit Cysteamin (= 2-Mercapto-ethylamin) umgesetzt, wobei man das 1-{4-[N-(2-Isopropyloxyethyl)-N-(2-aminoethylmercapto-acetyl)-amino]-benzoyl}-4-[2-(4-chlorphenyl)-ethyl]-piperazin erhält.

Beispiel 16: Setzt man in den Beispielen 1-15 anstelle der optisch aktiven Ausgangsmaterialien in der L-Form deren D-Formen oder Racemate ein, so erhält man die entsprechenden Enantiomere bzw. Racemate der dort erhaltenen Endstoffe.

Beispiel 17: Setzt man in den Beispielen 1-15 anstelle der optisch aktiven Ausgangsmaterialien in der D-Form deren L-Formen oder Racemate ein, so erhält man die entsprechenden Enantiomere bzw. Racemate der dort erhaltenen Endstoffe.

Beispiel 18: Setzt man in den Beispielen 1-15 anstelle der racemischen Ausgangsmaterialien deren L-Formen oder D-Formen ein, so erhält man die entsprechenden optischen Antipoden der dort erhaltenen Endstoffe.

Beispiel 19: Setzt man in den Beispielen 1-18 anstelle von 1-{4-[N-(2-Isopropyloxyethyl)-N-chloracetyl-amino]-benzoyl}-4-[2-(4-chlorphenyl)-ethyl]-piperazin-hydrochlorid das 1-{4-[N-(2-Hydroxyethyl)-N-chloracetyl-amino]-benzoyl}-4-[2-(4-chlorphenyl)-ethyl]-piperazin als Ausgangsmaterial ein, so erhält man in analoger Weise die folgenden Verbindungen:
(a) 1-{4-[N-(2-Hydroxyethyl)-N-([2R]-2-methoxycarbonyl-2-aminoethylmercaptoacetyl)-amino]-benzoyl}-4-[2-(4-chlorphenyl)-ethyl]-piperazin
(b) 1-{4[N-(2-Hydroxyethyl)-N-([2R]-2-carboxy-2-aminoethylmercapto-acetyl)-amino]-benzoyl}-4-[2-(4-chlorphenyl)-ethyl]-piperazin
(c) 1-{4-[N-(2-Hydroxyethyl)-N-([2R]-2-ethoxycarbonyl-2-aminoethylmercapto-acetyl)-amino]-benzoyl}-4-[2-(4-chlorphenyl)-ethyl]-piperazin
(d) 1-{4-[N-(2-Hydroxyethyl)-N([2R]-2-carboxy-2-N'-acetylamino-1,1-dimethyl-ethylmercapto-acetyl)-amino]-benzoyl}-4-[2-(4-chlorphenyl)-ethyl]-piperazin
(e) 1-{4-[N-(2-Hydroxyethyl)-N-([2R]-2-methoxycarbonyl-2-N'-acetylamin-1,1-dimethyl-ethylmercapto-acetyl)-amino]-benzoyl}-4-[2-(4-chlorphenyl)-ethyl]-piperazin
(f) 1-{4-[N-(2-Hydroxyethyl)-N-([2R]-2-ethoxycarbonyl-2-N'-acetylamino-1,1-dimethylethylmercapto-acetyl)-amino]-benzoyl}-4-[2-(4-chlorphenyl)-ethyl]-piperazin
(g) (±)-1-{4-[N-(2-Hydroxyethyl)-N-(2-carboxy-2-amino-1,1-dimethyl-ethylmercaptoacetyl)-amino]-benzoyl}-4-[2-(4-chlorphenyl)-ethyl]-piperazin
(h) (±)-1-{4-[N-(2-Hydroxyethyl)-N-(2-methoxycarbonyl-2-amino-1,1-dimethyl-ethylmercapto-acetyl)-amino]-benzoyl}-4-[2-(4-chlorphenyl)-ethyl]-piperazin
(i) (±)-1-{4-[N-(2-Hydroxyethyl)-N-(2-ethoxycarbonyl-2-amino-1,1-dimethyl-ethylmercapto-acetyl)-amino]-benzoyl}-4-[2-(4-chlorphenyl)-ethyl]-piperazin
(j) (±)-1-{4-[N-(2-Hydroxyethyl)-N-(1,2-dicarboxyethylmercapto-acetyl)-amino]-benzoyl}-4-[2-(4-chlorphenyl)-ethyl]-piperazin
(k) (±)-1-{4-[N-(2-Hydroxyethyl)-N-(1,2-di-methoxycarbonyl-ethylmercapto-acetyl)-amino]-benzoyl}-4-[2-(4-chlorphenyl)-ethyl]-piperazin
(1) (±)-1-{4-[N-(2-Hydroxyethyl)-N-(1,2-di-ethoxycarbonyl-ethylmercapto-acetyl)-amino]-benzoyl}-4-[2-(4-chlorphenyl)-ethyl]-piperazin
(m) 1-{4-[N-(2-Hydroxyethyl)-N-([2R]-2-carboxy-2-N'-acetylamino-ethylmercapto-acetyl)-amino]-benzoyl}-4-[2-(4-chlorphenyl)-ethyl]-piperazin
(n) 1-{4-[N-(2-Hydroxyethyl)-N-([2R]-2-methoxycarbonyl-2-N'-acetylamino-ethylmercapto-acetyl)-amino]-benzoyl}-4-[2-(4-chlorphenyl)-ethyl]-piperazin
(o) 1-{4-[N-(2-Hydroxyethyl)-N-([2R]-2-ethoxycarbonyl-2-N'-acetylamino-ethylmercapto-acetyl)-amino]-benzoyl}-4-[2-(4-chlorphenyl)-ethyl]-piperazin
(p) 1-{4-[N-(2-Hydroxyethyl)-N-(2-methoxycarbonyl-ethylmercapto-acetyl)-amino]-benzoyl}-4-[2-(4-chlorphenyl)-ethyl]-piperazin
(q) 1-{4-[N-(2-Hydroxyethyl)-N-(2-carboxy-ethylmercapto-acetyl)-amino]-benzoyl}-4-[2-(4-chlorphenyl)-ethyl]-piperazin
(r) 1-{4-[N-(2-Hydroxyethyl)-N-(2-carboxy-2-oxo-ethylmercapto-acetyl)-amino]-benzoyl}-4-[2-(4-chlorphenyl)-ethyl]-piperazin
(s) 1-{4-[N-(2-Hydroxyethyl)-N-(2-methoxycarbonyl-2-oxo-ethylmercapto-acetyl)-amino]-benzoyl}-4-[2-(4-chlorphenyl)-ethyl]-piperazin
(t) 1-{4-[N-(2-Hydroxyethyl)-N-(2-ethoxycarbonyl-2-oxo-ethylmercapto-acetyl)-amino]-benzoyl}-4-[2-(4-chlorphenyl)-ethyl]-piperazin
(u) 1-{{4-{N-(2-Hydroxyethyl)-N-[[[2R]-2-[N'-(carboxymethyl)carbamoyl]-2-[N''-(γ-L-glutamyl)amino]-ethylmercapto-acetyl]]-amino}-benzoyl}}-4-[2-(4-chlorphenyl)-ethyl]-piperazin. Das Dihydrochlorid-monohydrat davon hat einen Smp. von 140-143° und ein α_{D}²⁵= -13.3° (c = 0.768%, Methanol).
(v)(±)-1-{4-[N-(2-Hydroxyethyl)-N-(3-carboxy-3-amino-propylmercapto-acetyl)-amino]-benzoyl}-4-[2-(4-chlorphenyl)-ethyl]-piperazin
(w) (±)-1-{4-[N-(2-Hydroxyethyl)-N-(3-methoxycarbonyl-3-amino-propylmercapto-acetyl)-amino]-benzoyl}-4-[2-(4-chlorphenyl)-ethyl]-piperazin
(x) (±)-1-{4-[N-(2-Hydroxyethyl)-N-(3-ethoxycarbonyl-3-amino-propylmercapto-acetyl)-amino]-benzoyl}-4-[2-(4-chlorphenyl)-ethyl]-piperazin
(y) 1-{{4-{N-(2-Hydroxyethyl)-N-[[[2R]-2-[N'-(carboxymethyl)carbamoyl]-2-aminoethylmercapto-acetyl]]-amino}-benzoyl}}-4-[2-(4-chlorphenyl)-ethyl]-piperazin
(z)1-{4-[N-(2-Hydroxyethyl)-N-([2S]-2-methoxycarbonyl-2-aminoethylmercaptoacetyl)-amino]-benzoyl}-4-[2-(4-chlorphenyl)-ethyl]-piperazin
(aa) 1-{{4-{N-(2-Hydroxyethyl)-N-[[[2R]-2-[N'-(carboxymethyl)carbamoyl]-2-[N''-(γ-N'''-acetyl-L-glutamyl)amino]-ethylmercaptoacetyl]]amino]-benzoyl}}-4-[2-(4-chlorphenyl)-ethyl]-piperazin
(ab) 1-{4-[N-(2-Hydroxyethyl)-N-(methoxycarbonyl-methylmercapto-acetyl)-amino]-benzoyl}-4-[2-(4-chlorphenyl)-ethyl]-piperazin
(ac) 1-{4-[N-(2-Hydroxyethyl)-N-(3-carboxy-propylmercapto-acetyl)-amino]-benzoyl}-4-[2-(4-chlorphenyl)-ethyl]-piperazin
(ad) 1-{4-[N-(2-Hydroxyethyl)-N-(2-minoethylmercapto-acetyl)-amino]-benzoyl}-4-[2-(4-chlorphenyl)-ethyl]-piperazin.

Das Ausgangsmaterial wird wie folgt hergestellt:

### Ausgangsmaterial: 1-{4-[N-(2-Hydroxyethyl)-N-chloracetyl-amino]-benzoyl}-4-[2-(4-chlorphenyl)-ethyl]-piperazin

4.9 g 1-{4-[N-(2-Chloracetoxy-ethyl)-N-chloracetyl-amino]-benzoyl}-4-[2-(4-chlorphenyl)-ethyl]-piperazin werden in 30 ml Ethanol gelöst, mit 30 ml Wasser versetzt und mit etwa 2.5 ml konz. Ammoniak auf pH 9.5 gestellt. Nach 2h Rühren bei RT wird im Vakuum das Ethanol entfernt und die wässrige Lösung mit Methylenchlorid ausgeschüttelt, getrocknet und eingedampft. Das dunkle Öl wird über 150 g Kieselgel chromatographiert und das Eluat mit Methylenchlorid-Methanol 100:4 aus Isopropanol-Ether kristallisiert. Man erhält so das Ausgangsmaterial, Smp. 123-125°.

Die Ausgangsverbindung wird wie folgt hergestellt:
6 g 1-(4-Aminobenzoyl)-4-[2-(4-chlorphenyl)ethyl]-piperazin (s. EP-A-489 690, Bsp. 3), 2.4 g 2-Bromethanol und 0.6 g 4-Dimethylaminopyridin werden in 80 ml Isopropanol suspendiert und 22h am Rückfluss gekocht, danach eingedampft und zwischen Methylenchlorid und 1N Natronlauge verteilt. Die organische Phase wird mit Wasser gewaschen, getrocknet und eingedampft. Das ölige Material wird über 150 g Kieselgel chromatographiert. Durch Elution mit Methylenchlorid-Methanol (100:4 und 100:5) erhält man das 1-[4-N-(2-Hydroxyethyl)-amino]-benzoyl]-4-[2-(4-chlorphenyl)-ethyl]-piperazin, Smp. 94-95°.

3.4 g 1-[4-N-(2-Hydroxyethyl)-amino]-benzoyl]-4-[2-(4-chlorphenyl)-ethyl]-piperazin werden in 80 ml Methylenchlorid gelöst und zusammen mit 2.4 g Hünig-Base (= N-Ethyldiisopropylamin) vorgelegt. Dazu tropft man eine Lösung von 2.1 g Chloracetylchlorid in 15 ml Methylenchlorid innerhalb von 30 min zu. Nach 3.5h Rühren bei RT wird die dunkle Lösung mit Wasser neutral gewaschen, getrocknet und eingedampft. Man erhält so das ölige 1-{4-[N-(2-Chloracetoxy-ethyl)-N-chloracetyl-amino]-benzoyl}-4-[2-(4-chlorphenyl)-ethyl]-piperazin, welches ohne Reinigung weiter umgesetzt wird.

Das Ausgangsmaterial, 1-{4-[N-(2-Hydroxyethyl)-N-chloracetyl-amino]-benzoyl}-4-[2-(4-chlorphenyl)-ethyl]-piperazin, wirkt ebenso wie die Verbindungen der Formel I als Hemmer auf die Biosynthese von Interleukin-1 und kann in gleicher Weise wie für die Verbindungen der Formel I angegeben zur Behandlung der oben erwähnten Krankheitsbilder verwendet werden. Daher bildet diese Verbindung einschliesslich ihrer Salze, pharmazeutische Präparate, die sie enthalten, sowie ihre Verwendung und Herstellung einen weiteren Gegenstand der Erfindung.

Beispiel 20: Setzt man in den Beispielen 1-18 anstelle von 1-{4-[N-(2-Isopropyloxyethyl)-N-chloracetyl-amino]-benzoyl}-4-[2-(4-chlorphenyl)ethyl]-piperazin-hydrochlorid das 1-(4-N-Chloracetylaminobenzoyl)-4-[2-(4-chlorphenyl)-ethyl]-piperazin als Ausgangsmaterial ein, so erhält man in analoger Weise die folgenden Verbindungen:
(a) 1-{4-[N-([2R]-2-methoxycarbonyl-2-aminoethylmercapto-acetyl)-amino]-benzoyl}-4-[2-(4-chlorphenyl)-ethyl]-piperazin
(b) 1-{4-[N-([2R]-2-carboxy-2-aminoethylmercapto-acetyl)-amino]-benzoyl}-4-[2-(4-chlorphenyl)-ethyl]-piperazin
(c) 1-{4-[N-([2R]-2-ethoxycarbonyl-2-aminoethylmercapto-acetyl)-amino]-benzoyl}-4-[2-(4-chlorphenyl)-ethyl]-piperazin
(d) 1-{4-[N-([2R]-2-carboxy-2-N'-acetylamino-1,1-dimethyl-ethylmercapto-acetyl)-amino]-benzoyl}-4-[2-(4-chlorphenyl)-ethyl]-piperazin
(e) 1-{4-[N-([2R]-2-methoxycarbonyl-2-N'-acetylamino-1,1-dimethyl-ethylmercapto-acetyl)-amino]-benzoyl}-4-[2-(4-chlorphenyl)-ethyl]-piperazin
(f) 1-{4-[N-([2R]-2-ethoxycarbonyl-2-N'-acetylamino-1,1-dimethyl-ethylmercapto-acetyl)-amino]-benzoyl}-4-[2-(4-chlorphenyl)-ethyl]-piperazin
(g) (±)-1-{4-[N-(2-carboxy-2-amino-1,1-dimethyl-ethylmercapto-acetyl)-amino]-benzoyl}-4-[2-(4-chlorphenyl)-ethyl]-piperazin
(h) (±)-1-{4-[N-(2-methoxycarbonyl-2-amino-1,1-dimethyl-ethylmercapto-acetyl)-amino]-benzoyl}-4-[2-(4-chlorphenyl)-ethyl]-piperazin
(i) (±)-1-{4-[N-(2-ethoxycarbonyl-2-amino-1,1-dimethyl-ethylmercapto-acetyl)-amino]-benzoyl}-4-[2-(4-chlorphenyl)-ethyl]-piperazin
(j) (±)-1-{4-[N-(1,2-dicarboxyethylmercapto-acetyl)-amino]-benzoyl}-4-[2-(4-chlorphenyl)-ethyl]-piperazin
(k) (±)-1-{4-[N-(1,2-di-methoxycarbonyl-ethylmercapto-acetyl)-amino]-benzoyl}-4-[2-(4-chlorphenyl)-ethyl]-piperazin
(l) (±)-1-{4-[N-(1,2-di-ethoxycarbonyl-ethylmercapto-acetyl)-amino]-benzoyl}-4-[2-(4-chlorphenyl)-ethyl]-piperazin
(m) 1-{4-[N-([2R]-2-carboxy-2-N'-acetylamino-ethylmercapto-acetyl)-amino]-benzoyl}-4-[2-(4-chlorphenyl)-ethyl]-piperazin
(n) 1-{4-[N-([2R]-2-methoxycarbonyl-2-N'-acetylamino-ethylmercapto-acetyl)-amino]-benzoyl}-4-[2-(4-chlorphenyl)-ethyl]-piperazin
(o) 1-{4-[N-([2R]-2-ethoxycarbonyl-2-N'-acetylamino-ethylmercapto-acetyl)-amino]-benzoyl}-4-[2-(4-chlorphenyl)-ethyl]-piperazin
(p) 1-{4-[N-(2-methoxycarbonyl-ethylmercapto-acetyl)-amino]-benzoyl}-4-[2-(4-chlorphenyl)-ethyl]-piperazin
(q) 1-{4-[N-(2-carboxy-ethylmercapto-acetyl)-amino]-benzoyl}-4-[2-(4-chlorphenyl)-ethyl]-piperazin
(r) 1-{4-[N-(2-carboxy-2-oxo-ethylmercapto-acetyl)-amino]-benzoyl}-4-[2-(4-chlorphenyl)-ethyl]-piperazin
(s) 1-{4-[N-(2-methoxycarbonyl-2-oxo-ethylmercapto-acetyl)-amino]-benzoyl}-4-[2-(4-chlorphenyl)-ethyl]-piperazin
(t) 1-{4-[N-(2-ethoxycarbonyl-2-oxo-ethylmercapto-acetyl)amino]-benzoyl}-4-[2-(4-chlorphenyl)-ethyl]-piperazin
(u) 1-{{4-{N-[[[2R]-2-[N'-(carboxymethyl)carbamoyl]-2-[N''-(γ-L-glutamyl)amino]-ethylmercapto-acetyl]]amino}-benzoyl}}-4-[2-(4-chlorphenyl)-ethyl]-piperazin
(v) (±)-1-{4-[N-(3-carboxy-3-amino-propylmercapto-acetyl)-amino]-benzoyl}-4-[2-(4-chlorphenyl)-ethyl]-piperazin
(w) (±)-1-{4-[N-(3-methoxycarbonyl-3-amino-propylmercapto-acetyl)-amino]-benzoyl}-4-[2-(4-chlorphenyl)-ethyl]-piperazin
(x) (±)-1-{4-[N-(3-ethoxycarbonyl-3-amino-propylmercapto-acetyl)-amino]-benzoyl}-4-[2-(4-chlorphenyl)-ethyl]-piperazin
(y) 1-{{4-{N-[[[2R]-2-[N'-(carboxymethyl)carbamoyl]-2-amino-ethylmercaptoacetyl]]-amino}-benzoyl}}-4-[2-(4-chlorphenyl)-ethyl]-piperazin
(z) 1-{4-[N-([2S]-2-methoxycarbonyl-2-aminoethylmercapto-acetyl)-amino]-benzoyl}-4-[2-(4-chlorphenyl)-ethyl]-piperazin
(aa) 1-{{4-{N-[[[2R]-2-[N'-(carboxymethyl)carbamoyl]-2-[N''-(γ-N'''-acetyl-L-glutamyl)amino]-ethylmercapto-acetyl]]-amino}-benzoyl}}-4-[2-(4-chlorphenyl)-ethyl]-piperazin
(ab) 1-{4-[N-(methoxycarbonyl-methylmercapto-acetyl)-amino]-benzoyl}-4-[2-(4-chlorphenyl)-ethyl]-piperazin
(ac) 1-{4-[N-(3-carboxy-propylmercapto-acetyl)-amino]-benzoyl]-4-[2-(4-chlorphenyl)-ethyl]-piperazin
(ad) 1-{4-[N-(2-aminoethylmercapto-acetyl)-amino]-benzoyl}-4-[2-(4-chlorphenyl)-ethyl]-piperazin.

Das Ausgangsmaterial, 1-(4-N-Chloracetylaminobenzoyl)-4-[2-(4-chlorphenyl)-ethyl]-piperazin, wird wie folgt hergestellt:
1.7 g 1-(4-Aminobenzoyl)-4-[2-(4-chlorphenyl)-ethyl]-piperazin (s. EP-A-489 690, Bsp. 3) und 0.55 g Triethylamin werden in 25 ml Methylenchlorid vorgelegt. Dazu wird eine Lösung von 0.6 g Chloracetylchlorid zugetropft. Die dunkle Lösung wird über Nacht stehen gelassen, mit 50 ml Methylenchlorid verdünnt, mit 1 N Natronlauge gewaschen, über Magnesiumsulfat getrocknet und eingedampft. Das so erhaltene Kristallisat wird aus Aceton umkristallisiert. Man erhält so das Ausgangsmaterial, Smp. 146-147°.

Beispiel 21: 3.0 g 1-{-4-[N-(2-Isopropyloxyethyl)-N-chloracetyl-amino]-benzoyl}-4-[2-(4-chlorphenyl)-ethyl]-piperazin werden in 40 ml Wasser und 30 ml Ether suspendiert und vorgelegt. Man gibt unter Rühren 0.93 g L-Cystein-hydrochlorid-hydrat (Fa. Fluka) hinzu; dann werden innerhalb von 5 Minuten 3 ml konz. wässrige Ammoniak-Lösung hinzugetropft. Es ensteht eine milchige Emulsion, die innerhalb kurzer Zeit Klar wird. Nach 1h starkem Rühren wird die zweiphasige Reaktionsmischung in einen Scheidetrichter umgegossen. Die wässrige Phase wird abgetrennt, mit Ether gewaschen und im Vakuum eingedampft. Das schaumige Material wird in 20 ml Wasser gelöst und auf Antecgel-Dodecyltrichlorsilan (OPTI-UP C₁₂, Antec AG) chromatographiert [Elutionsmittel: (1) Wasser, um die organischen Salze auszuwaschen, und (2) Acetonitril-Wasser 4:6, um die Titelverbindung eluieren). Die vereinigten Fraktionen werden am Rotationsverdampfer eingedampft. Den erhaltenen Schaum in Methanol lösen und mit etherischer Salzsäure versetzen (pH 2), am Rotationsverdampfer eindampfen und gut trocknen. Man erhält so einen Schaum. Dieser wird mit Ether-Hexan verrührt, und man erhält eine Suspension, die abgenutscht wird. Das erhaltene farblose Produkt entspricht dem (-)-1-{4-[N-(2-Isopropyloxyethyl)-N-([2R]-2-carboxy-2-aminoethylmercapto-acetyl)-amino]-benzoyl}-4-[2-(4-chlorphenyl)-ethyl]-piperazin-hydrochlorid-trihydrat, Smp. 140-143°, α_{D}²⁵ = -23.0° (c = 1.01%, Methanol).

Beispiel 22: Aus dem Produkt von Beispiel 21 (Hydrochlorid) erhält man die freie Base wie folgt:
2.5 g Hydrochlorid in Wasser lösen, mit konz. Ammoniak auf pH 14 stellen, die milchige Lösung am Rotationsverdampfer eindampfen, den Rückstand in Wasser lösen und auf 50 g Antecgel-Dodecyltrichlorsilan (OPTI-UP C₁₂, Antec AG) auftragen, mit Wasser auswaschen, danach die freie Base mit 20%igem bzw. 40%igem wässrigem Acetonitril eluieren, das Eluat eindampfen, Rückstand mit Ether digerieren, abnutschen, mit Ether-Hexan waschen und trocknen. Man erhält so das farblose (-)-1-{4-[N-(2-Isopropyloxyethyl)-N-([2R]-2-carboxy-2-aminoethyl-mercapto-acetyl)-amino]-benzoyl}-4-[2-(4-chlorphenyl)-ethyl]-piperazin vom Smp. 91-92°; α_{D}²⁵= -25.2 (c = 0.59%, Methanol).

Beispiel 23: 3.5 g 1-{4-[N-(2-Isopropyloxyethyl)-N-([2R]-2-carboxy-2-N'-acetylamino-ethylmercapto-acetyl)-amino]benzoyl}-4-]2-(4-chlorphenyl)-ethyl]-piperazin (Beispiel 5) werden in 100 ml Methanol gelöst und mit 10 ml konz. Salzsäure in Ether versetzt. Die Lösung wird 2 h am Rückfluss erwärmt. Den Ether am Rotationsverdampfer entfernen, den öligen Rückstand in Essigester lösen und mit verdünnter Sodalösung waschen, trocknen und eindampfen. Da eine teilweise Spaltung der Acetylgruppe eingetreten ist, wird das ölige Produkt in 20 ml Essigester gelöst und mit 3 ml Acetanhydrid versetzt. Nach 2 h bei 80° wird das Gemisch am Rotationsverdampfer eingedampft, der Rückstand in 20 ml Dichloromethan gelöst und an Kieselgel chromatographiert (Elutionsmittel: Dichlormethan-Methanol 98:2). Das ölige Produkt wird in Methanol mit etherischer Salzsäure in 1-{4-[N-(2-Isopropyloxyethyl)-N-([2R]-2-methoxycarbonyl-2-N'-acetylamino-ethylmercapto-acetyl)-amino]-benzoyl}-4-[2-(4-chlorphenyl)-ethyl]-piperazin-hydrochlorid-hemihydrat vom Smp. 54-56° [α_{D}²⁵= -18.4°(c = 0.537%, Methanol)] überführt.

Beispiel 24: 2.1 g 1-{4-[N-(2-Isopropyloxyethyl)-N-([2R]-2-ethoxycarbonyl-2-aminoethylmercapto-acetyl)-amino]-benzoyl}-4-[2-(4-chlorphenyl)-ethyl]-piperazin (Beispiel 1b) werden in 20 ml Eisessig und 4 ml Acetanhydrid bei 70° acetyliert (1 h). Am Rotationsverdampfer eindampfen, den Rückstand in Methanol mit etherischer Salzsäure versetzen, wieder eindampfen und den Rückstand mit Hexan verrühren. Man erhält so das 1-{4-[N-(2-Isopropyloxyethyl)-N-([2R]-2-ethoxycarbonyl-2-N'-acetylamino-ethylmercapto-acetyl)-amino]-benzoyl}-4-[2-(4-chlorphenyl)-ethyl]-piperazinhydrochlorid-monohydrat vom Smp. 44-46°, α_{D}²⁵= -19.1° (c = 0.618%, Methanol).

Beispiel 25: 3 g 1-{4-[N-(2-Isopropyloxyethyl)-N-chloracetyl-amino]-benzoyl}-4-[2-(4-chlorphenyl)-ethyl]-piperazin werden in 50 ml Ether und 50 ml Wasser suspendiert und vorgelegt. Man gibt 1.69 g L-Glutathion (reduzierte Form, Fa. Fluka) [= γ-L-Glutamyl-L-cysteinyl-glycin] hinzu. Dann fügt man 3 ml konz. wässrige Ammoniak-Lösung zu. Es ensteht eine milchige Emulsion. Innerhalb von 5 Minuten bilden sich klare Schichten. Über Nacht wird das Gemisch stark gerührt. Die Etherphase wird abgetrennt und die wässrige Phase mit Ether gewaschen. Die vereinigten organischen Phasen werden mit Wasser gewaschen, getrocknet und eingedampft. Das ölige Material wird in Dichlormethan gelöst, mit konz. Ammoniak auf pH 14 gestellt und geschüttelt, getrocknet und eingedampft. Der Rückstand wird in 300 ml Essigester gelöst, über Magnesiumsulfat getrocknet und eingedampft. Der Rückstand wird mit Ether verrührt, abgenutscht und mit Ether gewaschen. Man erhält so das 1-{{4-{N-(2-Isopropyloxyethyl)-N-[[[2R]-2-[N'-(carboxymethyl)carbamoyl]-2-[N''-(γ-L-glutamyl)amino]-ethylmercapto-acetyl]]-amino}-benzoyl}}-4-[2-(4-chlorphenyl)-ethyl]-piperazin-trihydrat vom Smp. 169-173°, α_{D}²⁵= -18.4° (c = 1.01%, Methanol).

Beispiel 26: 2 g 1-{4-[N-(2-Isopropyloxyethyl)-N-chloracetyl-amino]-benzoyl}-4-[2-(4-chlorphenyl)-ethyl]-piperazin werden in 40 ml Wasser und 30 ml Ether suspendiert. Dazu gibt man 1.3 g L-Glutathionmonoethylester (Fa. Bachem) und 3 ml konz. Ammoniak. Es wird 2h bei Raumtemperatur gut gerührt, dann eingedampft und der Rückstand auf Antecgel-Dodecyltrichlorsilan (OPTI-UP C₁₂, Antec AG) chromatographiert. Mit zunehmendem Anteil von Acetonitril in Wasser wird (a) das 1-{{4-{N-(2-Isopropyloxyethyl)-N-[[[2R]-2-[N'-(carbamoylmethyl)carbamoyl]-2-[N''-(γ-L-glutamyl)amino]-ethylmercapto-acetyl]]-amino}-benzoyl}}-4-[2-(4-chlorphenyl)-ethyl]-piperazin-dihydrat [Smp. 120-122°, α_{D}²⁵= -8.3° (c = 0.577%, Methanol)] und (b) das 1-{{4-(N-(2-Isopropyloxyethyl)-N-[[[2R]-2-[N'-(ethoxycarbonylmethyl)carbamoyl]-2-[N''-(γ-L-glutamyl)amino]-ethylmercapto-acetyl]]-amino}-benzoyl}}-4-[2-(4-chlorphenyl)-ethyl]-piperazin-dihydrat [Smp. 95-97°, α_{D}²⁵= -20.8° (c = 0.495%, Methanol)] eluiert.

Beispiel 27: Führt man die Umsetzung des Beispiels 26 in Gegenwart von Hünig-Base anstelle von Ammoniak durch, so kann die Amidierung vermieden werden und man erhält ausschliesslich den Ethylester [Beispiel 26 (b)].

Beispiel 28: 1.33 g 1-{{4-{N-(2-Tetrahydropyran-2-yloxyethyl)-N-[[[2R]-2-[N'-(carboxymethyl)carbamoyl]-2-[N''-(γ-L-glutamyl)amino]-ethylmercapto-acetyl]]-amino}-benzoyl}}-4-[2-(4-chlorphenyl)-ethyl]-piperazin werden in 30 ml Methanol und 4 ml 2 N Salzsäure 1 h gut gerührt. Mit 2 N Natriumhydroxid-Lösung wird auf pH 7 gestellt, eingedampft, der Rückstand in 20 ml Wasser gelöst und auf Antecgel-Dodecyltrichlorsilan (OPTI-UP C₁₂, Antec AG) chromatographiert (Elutionsmittel: 10% bzw. 20%iges wässriges Acetonitril). Das Eluat wird mit Ether verrührt, abgenutscht und mit Ether gewaschen. Man erhält so das 1-{{4-{N-(2-Hydroxyethyl)-N-[[[2R]-2-[N'-(carboxymethyl)carbamoyl]-2-[N''-(γ-L-glutamyl)amino]-ethylmercapto-acetyl]]-amino}-benzoyl}}-4-[2-(4-chlorphenyl)-ethyl]-piperazin-monohydrat vom Smp. 160-162°, α_{D}²⁵ = -16.0° (c = 0.512%, Methanol).

Das Ausgangsmaterial wird wie folgt hergestellt:
1-(4-Amino-benzoyl)-4-[2-(4-chlorphenyl)-ethyl]-piperazin wird in 150 ml Tetrahydrofuran gelöst und vorgelegt. Dazu gibt man 4.51 g Hünig-Base und anschliessend tropfenweise innerhalb von 5 Minuten 10.91 g Chlorameisensäurebenzylester (50%ige Toluollösung, Fa. Merck). Die Innentemperatur steigt auf ca. 28°. Nach 45 Minuten Rühren wird die Suspension am Rotationsverdampfer eingedampft, der Rückstand in Dichlormethan gelöst, mit Wasser gewaschen, getrocknet und eingedampft. Der Rückstand wird in 30 ml Dichlormethan gelöst, dann vorsichtig 200 ml Ether zugegeben. Das Kristallisat wird abgenutscht und mit Ether gewaschen. Man erhält so das 1-[4-(N-Benzyloxycarbonylamino-benzoyl)-4-[2-(4-chlorphenyl)-ethyl]-piperazin vom Smp. 149-150°.

8.5 g davon werden in 80 ml Dimethylsulfoxid gelöst und 1.34 g pulverisiertes KOH zugeben. Man lässt 30 min bei RT rühren, versetzt dann mit 4.48 g 2-Tetrahydropyran-2-yloxyethylbromid (DE 1 237 110, Merck) und hält 8h bei 60° IT. Dann werden nochmals 0.5 g pulv. KOH sowie 1.0 g Bromid zugegeben und das Reaktionsgemisch weitere 3 h bei 60° gehalten. Die Suspension wird auf 500 ml Eiswasser gegossen und mit 300 ml Essigester versetzt. Die organische Phase wird abgetrennt, mit Wasser und Sodalösung gewaschen, getrocknet und eingedampft. Der ölige Rückstand wird in 50 ml Dichlormethan gelöst und auf Kieselgel chromatographiert (Elutionsmittel: Wasser-Methanol 99:1 bzw. 98:2). Das als Schaum erhaltene alkylierte Produkt wird ohne weitere Reinigung weiter umgesetzt.

9.25 g davon werden in Methanol mit Pd/C 10% hydriert. Dabei wird zunächst die Benzylgruppe abgespalten und dann die intermediär entstehende Carbonsäure decarboxyliert. Die Suspension wird auf Hyflofilter filtriert und das Filtrat eingedampft. Das gelbe Öl wird in Dichlormethan gelöst und mit 1 N Natronlauge gewaschen, getrocknet und eingedampft.

7.36 g des gelben Öls werden in 100 ml Tetrahydrofuran gelöst und mit 2.61 g Hünig-Base versetzt. Bei 3° IT werden 2.11 g Chloracetylchlorid in 10 ml Tetrahydrofuran dazugetropft. Man lässt auf RT erwärmen und rührt 1h weiter. Die Suspension wird eingedampft, der Rückstand in Essigester gelöst und mit Wasser gewaschen. Die Essigester-Phase wird noch mit 15%iger Soda-Lösung gewaschen, getrocknet und eingedampft. Das braune Öl wird auf 90 g Kieselgel chromatographiert (Elutionsmittel: CH₂Cl₂-Methanol 97:3). Das erhaltene Öl wird in Dichlormethan gelöst und mit etherischer Salzsäure versetzt (pH 2). Es wird eingedampft und der Rückstand mit Ether verführt. Man erhält so das 1-{4-[N-(2-Tetrahydropyran-2-yloxyethyl)-N-chloracetyl]-amino-benzoyl}-4-[2-(4-chlorphenyl)-ethyl]-piperazin-hydrochlorid-hemihydrat vom Smp. 97-100°.

4.8 g davon werden in 40 ml Ether und 50 ml Wasser gelöst und wie im Beispiel 1 beschrieben mit 2.8 g L-Glutathion und 5 ml konz. Ammoniak zu 1-{{4-{N-(2-Tetra-hydropyran-2-yloxyethyl)-N-[[[2R]-2-[N'-(carboxymethyl)carbamoyl]-2-[N''-(γ-L-glutamyl)amino]-ethylmercapto-acetyl]]-amino}-benzoyl}}-4-[2-(4-chlorphenyl)-ethyl]-piperazin vom Smp. 130-133° umgesetzt.

Beispiel 29: Analog zu Beispiel 21 werden 2.52 g 1-{4-[N-(2-Isopropyloxyethyl)-N-chloracetyl-amino]-benzoyl}-4-[2-(4-chlorphenyl)-ethyl]-piperazin und 1.0 g D-Cystein-hydrochlorid (anstelle von L-Cystein-hydrochlorid-hydrat) umgesetzt. Nach der Chromatographie auf Antecgel-Dodecyltrichlorsilan (OPTI-UP C₁₂, Antec AG) [Elutionsmittel: (1) Wasser, (2) Acetonitril-Wasser 1:9 bis 4:6], erhält man das (+)-1-{4-[N-(2-Isopropyloxyethyl)-N-([2S]-2-carboxy-2-aminoethylmercapto-acetyl)-amino]-benzoyl}-4-[2-(4-chlorphenyl)-ethyl]-piperazin-monohydrat, Smp. 90-92°, α_{D}²⁵= + 29.5° (c = 0.572%, Methanol).

Beispiel 30: Tabletten, enthaltend je 50 mg (-)-1-{4-[N-(2-Isopropyloxyethyl)-N-(2-methoxycarbonyl-2-aminoethylmercapto-acetyl)-amino]-benzoyl}-4-[2-(4-chlorphenyl)-ethyl]-piperazin-dihydrochlorid-trihydrat, werden z.B. wie folgt hergestellt:

| Zusammensetzung (10000 Tabletten) | |
|---|---|
| Wirkstoff | 500,0g |
| Lactose | 500,0 g |
| Kartoffelstärke | 352,0 g |
| Gelatine | 8,0 g |
| Talk | 60,0 g |
| Magnesiumstearat | 10,0 g |
| Siliciumdioxid (hochdispers) | 20,0 g |
| Ethanol | q.s. |

Der Wirkstoff wird mit der Lactose und 292 g Kartoffelstärke vermischt, die Mischung mit einer ethanolischen Lösung der Gelatine befeuchtet und durch ein Sieb granuliert. Nach dem Trocknen mischt man den Rest der Kartoffelstärke, das Magnesiumstearat, das Talk und das Siliciumdioxid zu und presst das Gemisch zu Tabletten von je 145,0 mg Gewicht und 50,0 mg Wirkstoffgehalt, die gewünschtenfalls mit Teilkerben zur feineren Anpassung der Dosierung versehen sein können.

Beispiel 31: Gelatinesteckkapseln, enthaltend 100 mg Wirkstoff, z.B. (-)-1-{4-[N-(2-Isopropyloxyethyl)-N-(2-methoxycarbonyl-2-aminoethylmercapto-acetyl)amino]-benzoyl}-4-[2-(4-chlorphenyl)-ethyl]-piperazin-dihydrochlorid-trihydrat, werden wie folgt hergestellt:

| Zusammensetzung (für 1000 Kapseln) | |
|---|---|
| Wirkstoff | 100,0 g |
| Lactose | 250,0 g |
| mikrokristalline Zellulose | 30,0 g |
| Natriumlaurylsulfat | 2,0 g |
| Magnesiumstearat | 8,0 g. |

Das Natriumlaurylsulfat wird durch ein Sieb mit einer Maschenweite von 0,2 mm zu dem lyophilisierten Wirkstoff hinzugesiebt. Beide Komponenten werden innig vermischt. Dann wird zunächst die Lactose durch ein Sieb mit einer Maschenweite von 0,6 mm und dann die mikrokristalline Zellulose durch ein Sieb mit einer Maschenweite von 0,9 mm hinzugesiebt. Daraufhin wird erneut 10 Minuten innig gemischt. Zuletzt wird das Magnesiumstearat durch ein Sieb mit einer Maschenweite von 0,8 mm hinzugesiebt. Nach 3- minütigem weiteren Mischen werden je 390 mg der erhaltenen Formulierung in Gelatinesteckkapseln der Grösse 0 abgefüllt.

Beispiel 32: Lacktabletten, enthaltend je 100 mg Wirkstoff, z.B. (-)-1-{4-[N-(2-Isopropyloxyethyl)-N-(2-methoxycarbonyl-2-aminoethylmercapto-acetyl)-amino]-benzoyl}-4-[2-(4-chlorphenyl)-ethyl]-piperazin-dihydrochlorid-trihydrat, werden wie folgt hergestellt:

| Zusammensetzung (für 1000 Lacktabletten) | |
|---|---|
| Wirkstoff 100,0 g | |
| Lactose | 100,0 g |
| Maisstärke | 70,0 g |
| Talk | 8,5 g |
| Calciumstearat | 1,5 g |
| Hydroxypropylmethylcellulose | 2,36 g |
| Schellack | 0,64 g |
| Wasser | q.s. |
| Methylenchlorid | q.s. . |

Der Wirkstoff, die Lactose und 40 g der Maisstärke werden gemischt und mit einem Kleister, hergestellt aus 15 g Maisstärke und Wasser (unter Erwärmen), befeuchtet und granuliert. Das Granulat wird getrocknet, der Rest der Maisstärke, der Talk und das Calciumstearat zugegeben und mit dem Granulat vermischt. Das Gemisch wird zu Tabletten (Gewicht: 280 mg) verpresst und diese mit einer Lösung der Hydroxypropyl-methylcellulose und des Schellacks in Methylenchlorid lackiert; Endgewicht der Lacktablette: 283 mg.

Beispiel 33: Eine 0,2%-ige Injektions- oder Infusionslösung eines Wirkstoffs, z.B. (-)-1-{4-[N-(2-Isopropyloxyethyl)-N-(2-methoxycarbonyl-2-aminoethylmercapto-acetyl)-amino]-benzoyl}-4-[2-(4-chlorphenyl)ethyl]-piperazin-dihydrochlorid-trihydrat, werden werden wie folgt hergestellt:

| Zusammensetzung (für 1000 Ampullen) | |
|---|---|
| Wirkstoff | 5,0 g |
| Natriumchlorid | 22,5 g |
| Phosphatpuffer pH= 7.4 | 300,0 g |
| entmineralisiertes Wasser | ad 2500,0 ml |

Der Wirkstoff und das Natriumchlorid werden in 1000 ml Wasser gelöst und durch ein Mikrofilter filtriert. Man versetzt mit der Pufferlösung und füllt mit Wasser auf 2500 ml auf. Zur Herstellung von Dosiseinheitsformen werden je 1,0 oder 2,5 ml in Glasampullen abgefüllt, die dann je 2,0 bzw. 5,0 mg Wirkstoff enthalten.

Beispiel 34: In analoger Weise wie in Beispiel 30 bis 33 beschrieben, können auch pharmazeutische Präparate, die jeweils eine andere der in den Beispielen 1 bis 29 genannten Verbindungen enthalten, hergestellt werden.

## Patentansprüche

1. Verbindungen der Formel I, 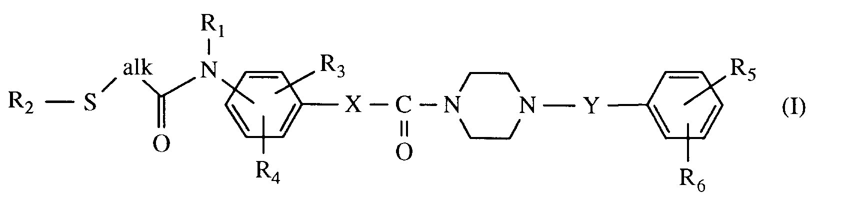 worin R₁ für Wasserstoff, Niederalkyl, Hydroxy-niederalkyl, Niederalkoxy-niederalkyl, Niederalkoxy-niederalkoxy-niederalkyl, Aryloxy-niederalkyl, Arylniederalkoxy-niederalkyl, N-Niederalkylamino-niederalkyl oder N,N-Diniederalkylamino-niederalkyl steht; R₂ Niederalkyl bedeutet, das durch einen Substituenten ausgewählt aus der Gruppe Amino, Acylamino, Carboxy und funktionell modifiziertes Carboxy substituiert ist und das gegebenenfalls weitere Substituenten ausgewählt aus der Gruppe Amino, Niederalkylamino, Diniederalkylamino, Acylamino, Oxo, Carboxy, funktionell modifiziertes Carboxy, Hydroxy, Niederalkoxy, Acyloxy und Halogen trägt; R₃ und R₄ unabhängig voneinander Wasserstoff, Niederalkyl, Halogen, Niederalkoxy oder Niederalkylthio bedeuten, R₅ und R₆ unabhängig voneinander für Wasserstoff, Niederalkyl, Halogen-niederalkyl, Niederalkoxy, Niederalkylthio, Halogen, Amino, Niederalkylamino, Diniederalkylamino oder Niederalkanoylamino stehen, alk Niederalkylen bedeutet, und X und Y unabhängig voneinander eine direkte Bindung, Niederalkylen oder Niederalkenylen bedeuten, und Salze davon.

2. Verbindungen der Formel I gemäss Anspruch 1, worin R₁ für Wasserstoff, Niederalkyl, Hydroxy-niederalkyl, Niederalkoxy-niederalkyl, Niederalkoxy-niederalkoxy-niederalkyl; Phenyloxy-niederalkyl oder Phenylniederalkoxy-niederalkyl, wobei in den beiden letztgenannten Resten die Phenylgruppe jeweils unsubstituiert oder durch Niederalkyl, Trifluormethyl, Niederalkoxy, Halogen und/oder Hydroxy substituiert ist; N-Niederalkylamino-niederalkyl oder N,N-Diniederalkylamino-niederalkyl steht; R₂ Niederalkyl bedeutet, das durch einen Substituenten ausgewählt aus der Gruppe gebildet aus Amino, Niederalkanoylamino, Carboxy, Niederalkoxycarbonyl, Carbamoyl, N-Niederalkylcarbamoyl, N,N-Diniederalkylcarbamoyl, Cyano und einem Rest -C(=O)-W₂, worin W₂ für das über eine Aminogruppe gebundene Radikal einer Aminosäure, oder ein Derivat davon, steht; substituiert ist und das gegebenenfalls weitere Substituenten ausgewählt aus der Gruppe gebildet aus Amino, Niederalkylamino, Diniederalkylamino, Niederalkanoylamino; einem Rest -NH-W₁, worin W₁ für das über eine beliebige Carboxygruppe gebundene Radikal einer Aminosäure, oder ein Derivat davon, steht; Oxo, Carboxy, Niederalkoxycarbonyl, Carbamoyl, N-Niederalkylcarbamoyl, N,N-Diniederalkylcarbamoyl, Cyano, Hydroxy, Niederalkoxy, Niederalkanoyloxy und Halogen trägt; R₃ und R₄ unabhängig voneinander Wasserstoff, Niederalkyl, Halogen, Niederalkoxy oder Niederalkylthio bedeuten, R₅ und R₆ unabhängig voneinander für Wasserstoff, Niederaikyl, Halogen-niederalkyl, Niederalkoxy, Niederalkylthio, Halogen, Amino, Niederalkylamino, Diniederalkylamino oder Niederalkanoylamino stehen, alk Niederalkylen bedeutet, und X und Y unabhängig voneinander eine direkte Bindung, Niederalkylen oder Niederalkenylen bedeuten, und Salze davon.

3. Verbindungen der Formel I gemäss Anspruch 1, worin R₁ für Wasserstoff, Niederalkyl, Hydroxy-niederaikyl, Niederalkoxy-niederalkyl, Niederalkoxy-niederalkoxy-niederalkyl, Phenyloxy-niederalkyl, N-Niederalkylamino-niederalkyl oder N,N-Diniederalkylamino-niederalkyl steht; R₂ Niederalkyl bedeutet, das durch einen Substituenten ausgewählt aus der Gruppe gebildet aus Amino, Carboxy, Niederalkoxycarbonyl, Carbamoyl, N-Niederalkylcarbamoyl, N,N-Diniederalkylcarbamoyl und einem Rest -C(=O)-W₂, worin W₂ für das über eine Aminogruppe gebundene Radikal einer natürlichen Aminosäure ausgewählt aus Glycin, Alanin, Valin, Leucin und Serin, oder einen Niederalkylester davon, steht; substituiert ist und das gegebenenfalls einen weiteren Substituenten ansgewählt aus der Gruppe gebildet aus Amino, Niederalkanoylamino; einem Rest -NH-W₁, worin W₁ für das über eine beliebige Carboxygruppe gebundene Radikal einer natürlichen Aminosäure ausgewählt aus Glutaminsäure, Asparaginsäure, Glycin, Alanin, Valin, Leucin und Serin, oder einen Niederalkylester davon, steht; Oxo, Carboxy, Niederalkoxycarbonyl, Carbamoyl, N,N-Diniederalkylcarbamoyl und Hydroxy trägt; R₃ und R₄ unabhängig voneinander Wasserstoff, Niederalkyl, Halogen, Niederalkoxy oder Niederalkylthio bedeuten, R₅ und R₆ unabhängig voneinander für Wasserstoff, Niederalkyl, Trifluormethyl, Niederalkoxy, Niederalkylthio, Halogen oder Diniederalkylamino stehen, alk Niederalkylen bedeutet, und X und Y unabhängig voneinander eine direkte Bindung, Niederalkylen oder Niederalkenylen bedeuten, und Salze davon.

4. Verbindungen der Formel I gemäss Anspruch 1, worin R₁ für Wasserstoff, Niederalkyl, Hydroxy-niederalkyl oder Niederalkoxy-niederalkyl steht, R₂ Niederalkyl bedeutet, das durch einen Substituenten ausgewählt aus der Gruppe gebildet aus Amino, Carboxy, Niederalkoxycarbonyl und einem Rest -C(=O)-W₂, worin W₂ für das über die Aminogruppe gebundene Radikal der Aminosäure Glycin, oder einen Niederalkylester davon, steht, substituiert ist und das gegebenenfalls einen weiteren Substituenten ausgewählt aus der Gruppe gebildet aus Amino, Niederalkanoylamino; einem Rest -NH-W₁, worin W₁ für das über die γ-Carboxygruppe gebundene Radikal der Aminosäure L-Glutaminsäure, oder einen Niederalkylester davon, steht; Oxo, Carboxy und Niederalkoxycarbonyl trägt; R₃ und R₄ unabhängig voneinander Wasserstoff, Niederalkyl, Halogen, Niederalkoxy oder Niederalkylthio bedeuten, R₅ für Niederalkylthio, Chlor, Fluor oder Brom steht, R₆ Wasserstoff bedeutet, alk Methylen bedeutet, X für eine direkte Bindung oder 1,2-Ethenylen steht, und Y 1,2-Ethylen bedeutet, und pharmazeutisch verwendbare Salze davon.

5. Verbindungen gemäss Anspruch 1 von der Formel Ia, 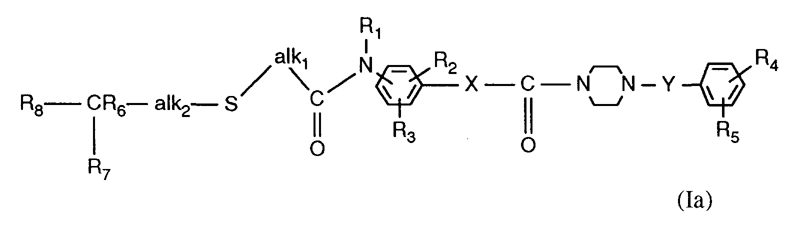 worin R₁ für Wasserstoff, Niederalkyl, Hydroxy-niederalkyl oder Niederalkoxyniederalkyl steht, R₂ und R₃ unabhängig voneinander Wasserstoff, Niederalkyl, Chlor, Fluor, Brom, Niederalkoxy oder Niederalkylthio bedeuten, R₄ und R₅ unabhängig voneinander Chlor, Fluor, Brom, Wasserstoff, Niederalkyl, Trifluormethyl, Niederalkoxy oder Niederalkylthio darstellen, R₆ für Wasserstoff steht, R₇ Wasserstoff, Amino, Niederalkanoylamino oder einen Rest -NH-W₁ bedeutet, worin W₁ für das über eine beliebige Carboxygruppe gebundene Radikal einer Aminosäure, oder ein Derivat davon, steht; oder R₆ und R₇ zusammen für Oxo stehen; R₈ Amino, Niederalkanoylamino, Carboxy, Niederalkoxycarbonyl, Carbamoyl, N-Niederalkylcarbamoyl, N,N-Diniederalkylcarbamoyl, Cyano oder einen Rest -C(=O)-W₂ bedeutet, worin W₂ für das über eine Aminogruppe gebundene Radikal einer Aminosäure, oder eines Derivats davon, steht; alk₁ für Niederalkylen steht, alk₂ eine direkte Bindung oder Niederalkylen, das unsubstituiert oder durch Carboxy, Niederalkoxycarbonyl, Carbamoyl, N-Niederalkylcarbamoyl, N,N-Diniederalkylcarbamoyl oder Cyano substituiert ist, bedeutet, X für eine direkte Bindung oder 1,2-Ethenylen steht, und Y eine direkte Bindung, Niederalkylen oder 1,2-Ethenylen bedeutet, und Salze davon.

6. Verbindungen der Formel Ia gemäss Anspruch 5, worin R₁ für Wasserstoff Niederalkyl, Hydroxy-niederalkyl oder Niederalkoxyniederalkyl steht, R₂ und R₃ unabhängig voneinander Wasserstoff, Niederalkyl, Chlor, Fluor oder Brom bedeuten, R₄ Chlor, Fluor, Brom oder Niederalkylthio darstellt, R₅ Wasserstoff bedeutet, R₆ für Wasserstoff steht, R₇ Wasserstoff, Amino, Niederalkanoylamino oder einen Rest -NH-W₁ bedeutet, worin W₁ für das über eine beliebige Carboxygruppe gebundene Radikal einer natürlichen Aminosäure, oder eines Niederalkylesters davon, steht; oder R₆ und R₇ zusammen für Oxo stehen; R₈ Amino, Carboxy, Niederalkoxycarbonyl, Carbamoyl, N-Niederalkylcarbamoyl, N,N-Diniederalkylcarbamoyl oder einen Rest -C(=O)-W₂ bedeutet, worin W₂ für das über eine Aminogruppe gebundene Radikal einer natürlichen Aminosäure, oder eines Niederalkylesters davon, steht; alk₁ für Methylen steht, alk₂ eine direkte Bindung, Methylen, 1-Methyl-methylen, 1,1-Dimethyl-methylen, 1-(Carboxy oder Niederalkoxycarbonyl)-methylen oder 1,2-Ethylen bedeutet, X für eine direkte Bindung oder 1,2-Ethenylen steht, und Y 1,2-Ethylen bedeutet, und pharmazeutisch verwendbare Salze davon.

7. Verbindungen der Formel Ia gemäss Anspruch 5, worin R₁ Wasserstoff, C₁-C₄-Alkyl, Hydroxy-C₂-C₄-alkyl oder C₁-C₄-Alkoxy-C₂-C₄-alkyl bedeutet, R₂ und R₃ unabhängig voneinander Wasserstoff, Niederalkyl, Chlor, Fluor oder Brom bedeuten, R₄ in para-Stellung angeknüpft ist und Chlor, Fluor, Brom oder Niederalkylthio darstellt, R₅ Wasserstoff bedeutet, R₆ für Wasserstoff steht, R₇ Wasserstoff, Amino, Nieder-alkanoylamino oder einen Rest -NH-W₁ bedeutet, worin W₁ für das über eine beliebige Carboxygruppe gebundene Radikal einer natürlichen Aminosäure ausgewählt aus Glutaminsäure, Asparaginsäure, Glycin, Alanin, Valin, Leucin und Serin, oder eines Niederalkylesters davon, steht; oder R₆ und R₇ zusammen für Oxo stehen; R₈ Amino, Carboxy, Niederalkoxycarbonyl, Carbamoyl, N-Niederalkylcarbamoyl, N,N-Diniederalkylcarbamoyl oder einen Rest -C(=O)-W₂ bedeutet, worin W₂ für das über eine Aminogruppe gebundene Radikal einer natürlichen Aminosäure ausgewählt aus Glycin, Alanin, Valin, Leucin und Serin, oder eines Niederalkylesters davon, steht; alk₁ für Methylen steht, alk₂ eine direkte Bindung, Methylen, 1,1-Dimethyl-methylen, 1-(Carboxy oder Niederalkoxycarbonyl)-methylen oder 1,2-Ethylen bedeutet, X für eine direkte Bindung oder 1,2-Ethenylen steht, und Y 1,2-Ethylen bedeutet, und pharmazeutisch verwendbare Salze davon.

8. Verbindungen der Formel Ia gemäss Anspruch 5, worin R₁ Wasserstoff, Methyl, Ethyl, 2-Hydroxyethyl oder 2-Isopropyloxyethyl bedeutet, R₂ und R₃ unabhängig voneinander Wasserstoff, Methyl, Chlor oder Fluor bedeuten, R₄ in para-Stellung angeknüpft ist und Chlor oder Brom darstellt, R₅ Wasserstoff bedeutet, R₆ für Wasserstoff steht, R₇ Wasserstoff, Amino, Niederalkanoylamino oder einen Rest -NH-W₁ bedeutet, worin W₁ für das über die γ-Carboxygruppe gebundene Radikal der Aminosäure L-Glutaminsäure, oder eines Niederalkylesters davon, steht; oder R₆ und R₇ zusammen für Oxo stehen; R₈ Amino, Carboxy, Niederalkoxycarbonyl oder einen Rest -C(=O)-W₂ bedeutet, worin W₂ für das über die Aminogruppe gebundene Radikal der Aminosäure Glycin, oder eines Niederalkylesters davon, steht; alk₁ für Methylen steht, alk₂ eine direkte Bindung, Methylen, 1,1-Dimethyl-methylen, 1-(Carboxy oder Niederalkoxycarbonyl)-methylen oder 1,2-Ethylen bedeutet, X für eine direkte Bindung oder 1,2-Ethenylen steht, und Y 1,2-Ethylen bedeutet, und pharmazeutisch verwendbare Salze davon.

9. Verbindungen der Formel Ia gemäss einem der Ansprüche 5-8, worin R₇ Niederalkanoylamino bedeutet, und pharmazeutisch verwendbare Salze davon.

10. (-)-1-{4-[N-(2-Isopropyloxyethyl)-N-([2R]-2-methoxycarbonyl-2-aninoethylmercapto-acetyl)-amino]-benzoyl}-4-[2-(4-chlorphenyl)-ethyl]-piperazin gemäss Anspruch 1, oder ein pharmazeutisch anwendbares Salz davon.

11. 1-{4-[N-(2-Isopropyloxyethyl)-N-([2R]-2-carboxy-2-N'-acetylamino-ethylmercapto-acetyl)-amino]-benzoyl}-4-[2-(4-chlorphenyl)-ethyl]-piperazin gemäss Anspruch 1, oder ein pharmazeutisch anwendbares Salz davon.

12. Pharmazeutische Präparate enthaltend eine Verbindung gemäss einem der Ansprüche 1 bis 11 und mindestens ein pharmazeutisch verwendbares Trägermaterial.

13. Eine Verbindung gemäss einem der Ansprüche 1 bis 11 zur Anwendung in einem Verfahren zur therapeutischen Behandlung des tierischen oder menschlichen Körpers.

14. Eine Verbindung gemäss einem der Ansprüche 1 bis 11 zur Verwendung bei der Behandlung von Krankheiten, die auf eine Hemmung von Interleukin-1 ansprechen.

15. Verwendung einer Verbindung gemäss einem der Ansprüche 1 bis 11 zur Herstellung pharmazeutischer Präparate.

16. Verwendung einer Verbindung gemäss einem der Ansprüche 1 bis 11 zur Herstellung pharmazeutischer Präparate für die Behandlung von Krankheiten, die auf eine Hemmung von Interleukin-1 ansprechen.

17. Verfahren zur Herstellung einer Verbindung der Formel I gemäss Anspruch 1, dadurch gekennzeichnet, dass man
a) eine Verbindung der Formel II, 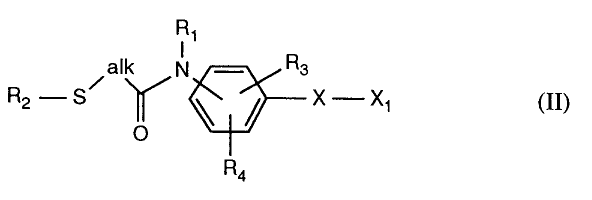 worin X₁ Carboxy oder reaktionsfähiges funktionell abgewandeltes Carboxy bedeutet, oder ein Salz davon, mit einer Verbindung der Formel III, 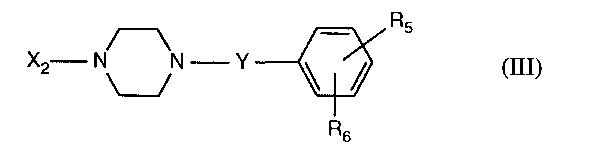 worin X₂ Wasserstoff oder eine Aminoschutzgruppe bedeutet, umsetzt, oder
b) eine Verbindung der Formel IV, 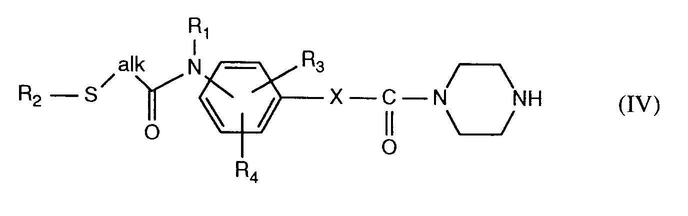 oder ein Salz davon, mit einer Verbindung der Formel V, 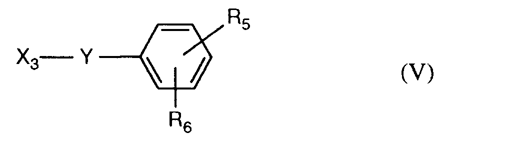 worin X₃ Hydroxy oder reaktionsfähiges verestertes Hydroxy bedeutet, umsetzt, oder
c) eine Verbindung der Formel VI, 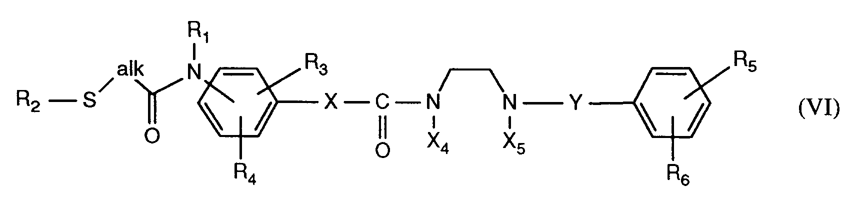 worin einer der Reste X₄ und X₅ Wasserstoff und der andere eine Gruppe der Formel -CH₂-CH₂-X₃ (VIa) bedeutet und X₃ für Hydroxy oder reaktionsfähiges verestertes Hydroxy steht, cyclisiert, oder
d) eine Verbindung der Formel VII, 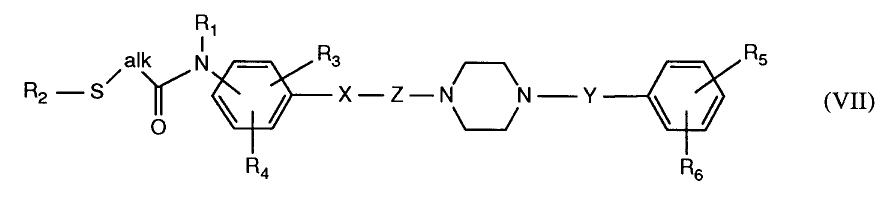 worin Z eine zu Carbonyl oxidierbare Gruppe bedeutet, oxidiert, oder
e) eine Verbindung der Formel VIII, 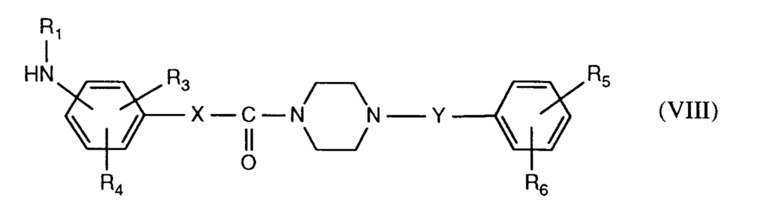 durch Umsetzung mit einer Verbindung der Formel IX, 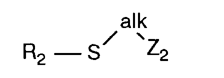 worin Z₂ Carboxy oder ein reaktionsfähiges Carboxyderivat bedeutet, acyliert, oder
f) eine Verbindung der Formel X, 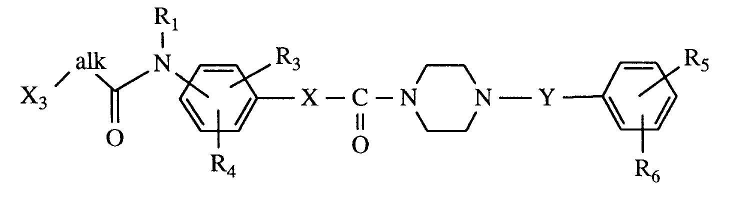 worin X₃ Hydroxy oder reaktionsfähiges verestertes Hydroxy bedeutet, mit einer Verbindung der Formel R₂-SH umsetzt;
und jeweils, wenn gewünscht, eine verfahrensgemäss oder auf andere Weise erhältliche Verbindung der Formel I in eine andere Verbindung der Formel I überführt, ein verfahrensgemäss erhältliches Isomerengemisch in die Komponenten auftrennt, eine vertahrensgemäss erhältliche freie Verbindung der Formel I in ein Salz überführt und/oder ein verfahrensgemäss erhältliches Salz in die freie Verbindung der Formel I oder in ein anderes Salz überführt.

18. 1-{4-[N-(2-Hydroxyethyl)-N-chloracetyl-amino]-benzoyl}-4-[2-(4-chlorphenyl)-ethyl]-piperazin, oder ein pharmazeutisch anwendbares Salz davon.
